# EUROPEAN PATENT SPECIFICATION

(11) **EP 1 427 826 B1**
(45) Date of publication and mention of the grant of the patent: **20.04.2011**
(21) Application number: 02798748.6
(22) Date of filing: 18.09.2002
(51) Int. Cl.: C12N 15/48

(54) **HIV- RT-nef-Gag codon optimised DNA vaccines**
Codon-optimisierte HIV-RT-nef-Gag DNA Impfstoffe
Vaccins VIH-RT-nef-Gag à DNA avec codons optimisés

(30) Priority: 20.09.2001 WO PCT/GB01/04207; 11.12.2001 GB 0129604; 19.03.2002 GB 0206462
(43) Date of publication of application: 16.06.2004
(60) Divisional application: 10180900.2
(73) Proprietor: GLAXO GROUP LIMITED, Greenford, Middlesex UB6 0NN (GB)
(72) Inventor: BEATON, Andrew, c/o GlaxoSmithKline, Stevenage, Hertfordshire SG1 2NY (GB); ERTL, Peter, Franz, c/o GlaxoSmithKline, Stevenage, Hertfordshire SG1 2NY (GB); GOUGH, Gerald, Wayne, c/o GlaxoSmithKline, Stevenage, Hertfordshire SG1 2NY (GB); LEAR, Andrew, c/o GlaxoSmithKline, Stevenage, Hertfordshire SG1 2NY (GB); TITE, John, Philip, c/o GlaxoSmithKline, Stevenage, Hertfordshire SG1 2NY (GB); VAN WELY, Catherine, Ann, c/o GlaxoSmithKline, Stevenage, Hertfordshire SG1 2NY (GB)
(74) Representative: Privett, Kathryn Louise
(86) International application number: PCT/EP2002/010592
(87) International publication number: WO 2003/025003

(56) References cited:
- WO-A-00/39302
- WO-A-01/43693
- WO-A-01/88141
- WO-A-02/32943
- WO-A-96/09378
- WO-A-97/31115
- WO-A-02/099101
- KOTSOPOULOU E ET AL: "A Rev-independent human immunodeficiency virus type 1 (HIV-1)-based vector that exploits a codon-optimized HIV-1 gag-pol gene" JOURNAL OF VIROLOGY, THE AMERICAN SOCIETY FOR MICROBIOLOGY, US, vol. 74, no. 10, May 2000 (2000-05), pages 4839-4852, XP002140792 ISSN: 0022-538X
- HUANG YUE ET AL: "Human immunodeficiency virus type 1-specific immunity after genetic immunization is enhanced by modification of Gag and Pol expression." JOURNAL OF VIROLOGY, vol. 75, no. 10, May 2001 (2001-05), pages 4947-4951, XP002237413 ISSN: 0022-538X
- GRAF M ET AL: "CONCERTED ACTION OF MULTIPLE CIS-ACTING SEQUENCES IS REQUIRED FOR REV DEPENDENCE OF LATE HUMAN IMMUNODEFICIENCY VIRUS TYPE 1 GENE EXPRESSION" JOURNAL OF VIROLOGY, THE AMERICAN SOCIETY FOR MICROBIOLOGY, US, vol. 74, no. 22, November 2000 (2000-11), pages 10822-10826, XP000971716 ISSN: 0022-538X
- SCHNEIDER R ET AL: "Inactivation of the human immunodeficiency virus type 1 inhibitory elements allows rev-independent expression of gag and gag/protease and particle formation" JOURNAL OF VIROLOGY, THE AMERICAN SOCIETY FOR MICROBIOLOGY, US, vol. 71, no. 7, July 1997 (1997-07), pages 4892-4903, XP002137891 ISSN: 0022-538X
- IGLESIAS E. ET AL: 'CHIMERIC PROTEINS CONTAINING HIV-1 T CELL EPITOPES: EXPRESSION IN E. COLI, PURIFICATION AND INDUCTION OF ANTIBODIES IN MICE' JOURNAL OF BIOCHEMISTRY, MOLECULAR BIOLOGY AND BIOPHYSICS, HARWOOD ACADEMIC PUBLISHERS, AMSTERDAM, NL vol. 5, 01 January 2001, pages 109 - 122, XP009035847 ISSN: 1025-8140
- PELEMANS ET AL MOLECULAR PHARMACOLOGY MAY 2000 vol. 57, no. 5, May 2000, pages 954 - 960 ISSN: 0026-895X
- WOODBERRY ET AL: 'Imunogenicity of a Human Immunodeficiency Virus (HIV) polytope vaccine containing multiple HLA2 HIV CD8+ cytotoxic T-cell epitopes' JOURNAL OF VIROLOGY vol. 73, no. 3, 1999, pages 5320 - 5325

## Description

### Field of the Invention

The present invention relates to nucleic acid constructs, host cells comprising such constructs and their use in nucleic acid vaccines. The invention further relates to vaccine formulations comprising such constructs and the use of such formulations in medicine. The invention in particular relates to DNA vaccines that are useful in the prophylaxis and treatment of HIV infections, more particularly when administered by particle mediated delivery.

### Background to the Invention

HIV-1 is the primary cause of the acquired immune deficiency syndrome (AIDS) which is regarded as one of the world's major health problems. Although extensive research throughout the world has been conducted to produce a vaccine, such efforts thus far have not been successful.

Non-envelope proteins of HIV-1 have been described and include for example internal structure proteins such as the products of the gag and pol genes and, other non-structural proteins such as Rev, Nef, Vif and Tat (Green et al., New England J. Med, 324, 5, 308 et seq (1991) and Bryant et al. (Ed. Pizzo), Pediatr. Infect. Dis. J., 11, 5, 390 et seq (1992).

The Gag gene is translated from the full-length RNA to yield a precursor polyprotein which is subsequently cleaved into 3 - 5 capsid proteins; the matrix protein, capsid protein and nucleic acid binding protein and protease. (1. Fundamental Virology, Fields BN, Knipe DM and Howley M 1996 2. Fields Virology vol 2 1996).

The gag gene gives rise to the 55-kilodalton (kD) Gag precursor protein, also called p55, which is expressed from the unspliced viral mRNA. During translation, the N terminus of p55 is myristoylated, triggering its association with the cytoplasmic aspect of cell membranes. The membrane-associated Gag polyprotein recruits two copies of the viral genomic RNA along with other viral and cellular proteins that triggers the budding of the viral particle from the surface of an infected cell. After budding, p55 is cleaved by the virally encoded protease (a product of the pol gene) during the process of viral maturation into four smaller proteins designated MA (matrix [p17]), CA (capsid [p24]), NC (nucleocapsid [p9]), and p6. (4)

In addition to the 3 major Gag protein, all Gag precursors contain several other regions, which are cleaved out and remain in the virion as peptides of various sizes. These proteins have different roles e.g. the p2 protein has a proposed role in regulating activity of the protease and contributes to the correct timing of proteolytic processing.

The MA polypeptide is derived from the N-terminal, myristoylated end of p55. Most MA molecules remain attached to the inner surface of the virion lipid bilayer, stabilizing the particle. A subset of MA is recruited inside the deeper layers of the virion where it becomes part of the complex which escorts the viral DNA to the nucleus. (5) These MA molecules facilitate the nuclear transport of the viral genome because a karyophilic signal on MA is recognized by the cellular nuclear import machinery. This phenomenon allows HIV to infect nondividing cells, an unusual property for a retrovirus.

The p24 (CA) protein forms the conical core of viral particles. Cyclophilin A has been demonstrated to interact with the p24 region of p55 leading to its incorporation into HIV particles. The interaction between Gag and cyclophilin A is essential because the disruption of this interaction by cyclosporine A inhibits viral replication.

The NC region of Gag is responsible for specifically recognizing the so-called packaging signal of HIV. The packaging signal consists of four stem loop structures located near the 5' end of the viral RNA, and is sufficient to mediate the incorporation of a heterologous RNA into HIV-1 virions. NC binds to the packaging signal through interactions mediated by two zinc-finger motifs. NC also facilitates reverse transcription.

The p6 polypeptide region mediates interactions between p55 Gag and the accessory protein Vpr, leading to the incorporation of Vpr into assembling virions. The p6 region also contains a so-called late domain which is required for the efficient release of budding virions from an infected cell

The Pol gene encodes two proteins containing the two activities needed by the virus in early infection, the RT and the integrase protein needed for integration of viral DNA into cell DNA. The primary product of Pol is cleaved by the virion protease to yield the amino terminal RT peptide which contains activities necessary for DNA synthesis (RNA and DNA directed DNA polymerase, ribouclease H) and carboxy terminal integrase protein.

HIV RT is a heterodimer of full-length RT (p66) and a cleavage product (p51) lacking the carboxy terminal Rnase integrase domain.

RT is one of the most highly conserved proteins encoded by the retroviral genome. Two major activities of RT are the DNA Pol and Ribonuclease H. The DNA Pol activity of RT uses RNA and DNA as templates interchangeably and like all DNA polymerases known is unable to initiate DNA synthesis de novo, but requires a pre existing molecule to serve as a primer (RNA).

The Rnase H activity inherent in all RT proteins plays the essential role early in replication of removing the RNA genome as DNA synthesis proceeds. It selectively degrades the RNA from all RNA - DNA hybrid molecules. Structurally the polymerase and ribo H occupy separate, non-overlapping domains with the Pol covering the amino two thirds of the Pol.

The p66 catalytic subunit is folded into 5 distinct subdomains. The amino terminal 23 of these have the portion with RT activity. Carboxy term to these is the Rnase H Domain.

After infection of the host cell, the retroviral RNA genome is copied into linear ds DNA by the reverse transcriptase that is present in the infecting particle. The integrase (reviewed in Skalka AM '99 Adv in Virus Res 52 271-273) recognises the ends of the viral DNA, trims them and accompanies the viral DNA to a host chromosomal site to catalyse integration. Many sites in the host DNA can be targets for integration. Although the integrase is sufficient to catalyse integration in vitro, it is not the only protein associated with the viral DNA in vivo - the large protein - viral DNA complex isolated from the infected cells has been denoted the pre integration complex. This facilitates the acquisition of the host cell genes by progeny viral genomes.

The integrase is made up of 3 distinct domains, the N terminal domain, the catalytic core and the c terjminal domain. The catalytic core domain contains all of the requirements for the chemistry of polynucleotidyl transfer.

The Nef protein is known to cause the removal of CD4, the HIV receptor, from the cell surface, but the biological importance of this function is debated. Additionally Nef interacts with the signal pathway of T cells and induces an active state, which in turn may promote more efficient gene expression. Some HIV isolates have mutations in this region, which cause them not to encode functional protein and are severely compromised in their replication and pathogenesis in vivo.

DNA vaccines usually consist of a bacterial plasmid vector into which is inserted a strong promoter, the gene of interest which encodes for an antigenic peptide and a polyadenylation/transcriptional termination sequences. The gene of interest may encode a full protein or simply an antigenic peptide sequence relating to the pathogen, tumour or other agent which is intended to be protected against. The plasmid can be grown in bacteria, such as for example E.coli and then isolated and prepared in an appropriate medium, depending upon the intended route of administration, before being administered to the host. Following administration the plasmid is taken up by cells of the host where the encoded peptide is produced. The plasmid vector will preferably be made without an origin of replication which is functional in eukaryotic cells, in order to prevent plasmid replication in the mammalian host and integration within chromosomal DNA of the animal concerned.

There are a number of advantages of DNA vaccination relative to traditional vaccination techniques. First, it is predicted that because of the proteins which are encoded by the DNA sequence are synthesised in the host, the structure or conformation of the protein will be similar to the native protein associated with the disease state. It is also likely that DNA vaccination will offer protection against different strains of a virus, by generating cytotoxic T lymphcyte response that recognise epitopes from conserved proteins. Furthermore, because the plasmids are taken up by the host cells where antigenic protein can be produced, a long-lasting immune response will be elicited. The technology also offers the possibility of combing diverse immunogens into a single preparation to facilitate simultaneous immunisation in relation to a number of disease states.

Helpful background information in relation to DNA vaccination is provided in Donnelly et al "DNA vaccines" Ann. Rev Immunol. 1997 15: 617-648, the disclosure of which is included herein in its entirety by way of reference.

Polytope DNA vaccines against HIV are described by Woodberry et al., journal of virology, 1999, vol. 73, No.7, p. 5320-5325

### Summary of the Invention

The present invention provides novel constructs for use in nucleic acid vaccines for the prophylaxis and treatment of HIV infections and AIDS, as defined in the claims.

There is disclosed a nucleic acid molecule comprising a nucleotide sequence encoding HIV gag protein or fragment thereof linked to a nucleotide sequence encoding a further HIV antigen or fragment thereof and operably linked to a heterologous promoter. The fragment of said nucleotide sequence will encode an HIV epitope and typically encode a peptide of at least 8 amino acids. The nucleotide sequence is preferably a DNA sequence and is preferably contained within a plasmid without an origin of replication. Such nucleic acid molecules are formulated with pharmaceutically acceptable excipient, carriers, diluents or adjuvants to produce pharmaceutical composition suitable for the treatment and/or prophylaxis of HIV infection and AIDS.

In a preferred embodiment the DNA sequence is formulated onto the surface of inert particles or beads suitable for particle mediated drug delivery. Preferably the beads are gold.

The invention provides a DNA sequence that highly expressed codes for gag protein which sequence is optimised to resemble the codon usage of genes in mammalian cells. In particular, the gag protein is optimised to resemble that of highly expressed human genes.

The DNA code has 4 letters (A, T, C and G) and uses these to spell three letter "codons" which represent the amino acids the proteins encoded in an organism's genes. The linear sequence of codons along the DNA molecule is translated into the linear sequence of amino acids in the protein(s) encoded by those genes. The code is highly degenerate, with 61 codons coding for the 20 natural amino acids and 3 codons representing "stop" signals. Thus, most amino acids are coded for by more than one codon - in fact several are coded for by four or more different codons.

Where more than one codon is available to code for a given amino acid, it has been observed that the codon usage patterns of organisms are highly non-random. Different species show a different bias in their codon selection and, furthermore, utilisation of codons may be markedly different in a single species between genes which are expressed at high and low levels. This bias is different in viruses, plants, bacteria and mammalian cells, and some species show a stronger bias away from a random codon selection than others. For example, humans and other mammals are less strongly biased than certain bacteria or viruses. For these reasons, there is a significant probability that a mammalian gene expressed in E.coli or a foreign or recombinant gene expressed in mammalian cells will have an inappropriate distribution of codons for efficient expression. It is believed that the presence in a heterologous DNA sequence of clusters of codons or an abundance of codons which are rarely observed in the host in which expression is to occur, is predictive of low heterologous expression levels in that host.

Disclosed is a gag polynucleotide sequence which encodes an amino acid sequence, wherein the codon usage pattern of the polynucleotide sequence resembles that of highly expressed mammalian genes. Preferably the polynucleotide sequence is a DNA sequence. Desirably the codon usage pattern of the polynucleotide sequence is typical of highly expressed human genes.

In the polynucleotides of the present invention, the codon usage pattern is altered from that typical of human immunodeficiency viruses to more closely represent the codon bias of the target organism, e.g. a mammal, especially a human. The "codon usage coefficient" is a measure of how closely the codon pattern of a given polynucleotide sequence resembles that of a target species. Codon frequencies can be derived from literature sources for the highly expressed genes of many species (see e.g. Nakamura et.al. Nucleic Acids Research 1996, 24:214-215). The codon frequencies for each of the 61 codons (expressed as the number of occurrences occurrence per 1000 codons of the selected class of genes) are normalised for each of the twenty natural amino acids, so that the value for the most frequently used codon for each amino acid is set to 1 and the frequencies for the less common codons are scaled to lie between zero and 1. Thus each of the 61 codons is assigned a value of 1 or lower for the highly expressed genes of the target species. In order to calculate a codon usage coefficient for a specific polynucleotide, relative to the highly expressed genes of that species, the scaled value for each codon of the specific polynucleotide are noted and the geometric mean of all these values is taken (by dividing the sum of the natural logs of these values by the total number of codons and take the anti-log). The coefficient will have a value between zero and 1 and the higher the coefficient the more codons in the polynucleotide are frequently used codons. If a polynucleotide sequence has a codon usage coefficient of 1, all of the codons are "most frequent" codons for highly expressed genes of the target species.

According to the present invention, the codon usage pattern of the polynucleotide will preferably exclude codons with an RSCU value of less than 0.2 in highly expressed genes of the target organism. Alternatively, the codon usage pattern will exclude codons representing <10% of the codons used for a particular amino acid. A relative synonymous codon usage (RSCU) value is the observed number of codons divided by the number expected if all codons for that amino acid were used equally frequently. A polynucleotide of the present invention will generally have a codon usage coefficient (or RSCU) for highly expressed human genes of greater than 0.3, preferably greater than 0.4, most preferably greater than 0.5 Codon usage tables for human can also be found in Genebank.

In comparison, a highly expressed beta actin gene has a RSCU of 0.747. The codon usage table for a homo sapiens is set out below:

### Codon Usage Table 1:

| ***Homo sapiens* [gbpri]: 27143 CDS's (12816923 codons)** | | | | | | | |
|---|---|---|---|---|---|---|---|
| fields: - [triplet] [frequency: **per thousand**] ([number]) | | | | | | | |
| UUU | 17.0(217684) | UCU | 14.8(189419) | UAU | 12.1(155645) | UGU | 10.0(127719) |
| UUC | 20.5(262753) | UCC | 17.5(224470) | UAC | 15.8(202481) | UGC | 12.3(157257) |
| UUA | 7.3( 93924) | UCA | 11.9(152074) | UAA | 0.7( 9195) | UGA | 1.3( 16025) |
| UUG | 12.5(159611) | UCG | 4.5( 57572) | UAG | 0.5( 6789) | UGG | 12.9(165930) |
| | | | | | | | |
| CUU | 12.8(163707) | CCU | 17.3(222146) | CAU | 10.5(134186) | CGU | 4.6( 59454) |
| CUC | 19.3(247391) | CCC | 20.0(256235) | CAC | 14.9(190928) | CGC | 10.8(137865) |
| CUA | 7.0( 89078) | CCA | 16.7(214583) | CAA | 12.0(153590) | CGA | 6.3( 80709) |
| CUG | 39.7(509096) | CCG | 7.0( 89619) | CAG | 34.5(441727) | CGG | 11.6(148666) |
| | | | | | | | |
| AUU | 15.8(202844) | ACU | 12.9(165392) | AAU | 17.0(218508) | AGU | 12.0(154442) |
| AUC | 21.6(277066) | ACC | 19.3(247805) | AAC | 19.8(253475) | AGC | 19.3(247583) |
| AUA | 7.2( 92133) | ACA | 14.9(191518) | AAA | 24.0(308123) | AGA | 11.5(147264) |
| AUG | 22.3(285776) | ACG | 6.3( 80369) | AAG | 32.6(418141) | AGG | 11.3(145276) |
| GUU | 10.9(139611) | GCU | 18.5(236639) | GAU | 22.4(286742) | GGU | 10.8(138606) |
| GUC | 14.6(187333) | GCC | 28.3(362086) | GAC | 26.1(334158) | GGC | 22.7(290904) |
| GUA | 7.0( 89644) | GCA | 15.9(203310) | GAA | 29.1(373151) | GGA | 16.4(210643) |
| GUG | 28.8(369006) | GCG | 7.5( 96455) | GAG | 40.2(515485) | GGG | 16.4(209907) |

Coding GC 52.51% 1st letter GC 56.04% 2nd letter GC 42.35% 3rd letter GC 59.13%

Codon Usage Table 2 (preferred):

| Codon usage for human (highly expressed) genes 1/24/91 (human_high.cod) | | | | |
|---|---|---|---|---|
| AmAcid | Codon | Number | /1000 | Fraction .. |
| Gly | GGG | 905.00 | 18.76 | 0.24 |
| Gly | GGA | 525.00 | 10.88 | 0.14 |
| Gly | GGT | 441.00 | 9.14 | 0.12 |
| Gly | GGC | 1867.00 | 38.70 | 0.50 |
| | | | | |
| Glu | GAG | 2420.00 | 50.16 | 0.75 |
| Glu | GAA | 792.00 | 16.42 | 0.25 |
| Asp | GAT | 592.00 | 12.27 | 0.25 |
| Asp | GAC | 1821.00 | 37.75 | 0.75 |
| | | | | |
| Val | GTG | 1866.00 | 38.68 | 0.64 |
| Val | GTA | 134.00 | 2.78 | 0.05 |
| Val | GTT | 198.00 | 4,10 | 0.07 |
| Val | GTC | 728.00 | 15.09 | 0.25 |
| | | | | |
| Ala | GCG | 652.00 | 13.51 | 0.17 |
| Ala | GCA | 488.00 | 10.12 | 0.13 |
| Ala | GCT | 654.00 | 13.56 | 0.17 |
| Ala | GCC | 2057.00 | 42.64 | 0.53 |
| | | | | |
| Arg | AGG | 512.00 | 10.61 | 0.18 |
| Arg | AGA | 298.00 | 6.18 | 0.10 |
| Ser | AGT | 354.00 | 7.34 | 0.10 |
| Ser | AGC | 1171.00 | 24.27 | 0.34 |
| | | | | |
| Lys | AAG | 2117.00 | 43.88 | 0.82 |
| Lys | AAA | 471.00 | 9.76 | 0.18 |
| Asn | AAT | 314.00 | 6.51 | 0.22 |
| Asn | AAC | 1120.00 | 23.22 | 0.78 |
| | | | | |
| Met | ATG | 1077.00 | 22.32 | 1.00 |
| Ile | ATA | 88.00 | 1.82 | 0.05 |
| Ile | ATT | 315.00 | 6.53 | 0.18 |
| Ile | ATC | 1369.00 | 28.38 | 0.77 |
| | | | | |
| Thr | ACG | 405.00 | 8.40 | 0.15 |
| Thr | ACA | 373.00 | 7.73 | 0.14 |
| Thr | ACT | 358.00 | 7.42 | 0.14 |
| Thr | ACC | 1502.00 | 31.13 | 0.57 |
| | | | | |
| Trp | TGG | 652.00 | 13.51 | 1.00 |
| End | TGA | 109.00 | 2.26 | 0.55 |
| Cys | TGT | 325.00 | 6.74 | 0.32 |
| Cys | TGC | 706.00 | 14.63 | 0.68 |
| | | | | |
| End | TAG | 42.00 | 0.87 | 0.21 |
| End | TAA | 46.00 | 0.95 | 0.23 |
| Tyr | TAT | 360.00 | 7.46 | 0.26 |
| Tyr | TAC | 1042.00 | 21.60 | 0.74 |
| | | | | |
| Leu | TTG | 313.00 | 6.49 | 0.06 |
| Leu | TTA | 76.00 | 1.58 | 0.02 |
| Phe | TTT | 336.00 | 6.96 | 0.20 |
| Phe | TTC | 1377.00 | 28.54 | 0.80 |
| | | | | |
| Ser | TCG | 325.00 | 6.74 | 0.09 |
| Ser | TCA | 165.00 | 3.42 | 0.05 |
| Ser | TCT | 450.00 | 9.33 | 0.13 |
| Ser | TCC | 958.00 | 19.86 | 0.28 |
| | | | | |
| Arg | CGG | 611.00 | 12.67 | 0.21 |
| Arg | CGA | 183.00 | 3.79 | 0.06 |
| Arg | CGT | 210.00 | 4.35 | 0.07 |
| Arg | CGC | 1086.00 | 22.51 | 0.37 |
| | | | | |
| Gln | CAG | 2020.00 | 41.87 | 0.88 |
| Gln | CAA | 283.00 | 5.87 | 0.12 |
| His | CAT | 234.00 | 4.85 | 0.21 |
| His | CAC | 870.00 | 18.03 | 0.79 |
| | | | | |
| Leu | CTG | 2884.00 | 59.78 | 0.58 |
| Leu | CTA | 166.00 | 3.44 | 0.03 |
| Leu | CTT | 238.00 | 4.93 | 0.05 |
| Leu | CTC | 1276.00 | 26.45 | 0.26 |
| | | | | |
| Pro | CCG | 482.00 | 9.99 | 0.17 |
| Pro | CCA | 456.00 | 9.45 | 0.16 |
| Pro | CCT | 568.00 | 11.77 | 0.19 |
| Pro | CCC | 1410.00 | 29.23 | 0.48 |

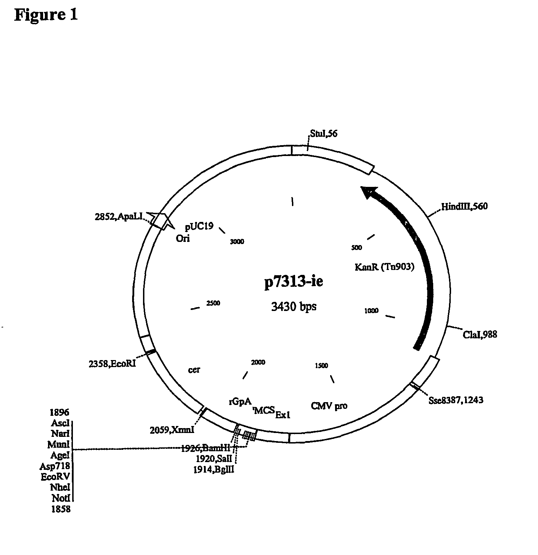

According to a further aspect of the invention, an expression vector is provided which comprises and is capable of directing the expression of a polynucleotide sequence according to the first aspect of the invention, in particular the codon usage pattern of the gag polynucleotide sequence is typical of highly expressed mammalian genes, preferably highly expressed human genes. The vector may be suitable for driving expression of heterologous DNA in bacterial insect or mammalian cells, particularly human cells. In one embodiment, the expression vector is p7313 (see figure 1).

In a preferred embodiment, the gag gene does not encode the gag p6 peptide. The NEF gene is truncated to remove the sequence encoding the N terminal region i.e. removal of 30-85, preferably 60-85, typically about 81, preferably the N terminal 65 amino acids.

In the invention the RT gene is also optimised to resemble a highly expressed human gene. The RT preferably encodes a mutation to substantially inactivate any reverse transcriptase activity. A preferred inactivation mutation involves the substitution of W tryptophan 229 for K (lysine).

According to a further aspect of the invention, a host cell comprising a polynucleotide sequence according to the invention, or an expression vector according to the invention is provided. The host cell may be bacterial, e.g. E.coli, mammalian, e.g. human, or may be an insect cell. Mammalian cells comprising a vector according to the present invention may be cultured cells transfected in vitro or may be transfected in vivo by administration of the vector to the mammal.

The present invention further provides a pharmaceutical composition comprising a polynucleotide sequence according to the invention. Preferably the composition comprises a DNA vector. In preferred embodiments the composition comprises a plurality of particles, preferably gold particles, coated with DNA comprising a vector encoding a polynucleotide sequence of the invention. Preferably the sequence encodes an HIV gag amino acid sequence, wherein the codon usage pattern of the polynucleotide sequence is typical of highly expressed mammalian genes, particularly human genes. In alternative embodiments, the composition comprises a pharmaceutically acceptable excipient and a DNA vector according to the second aspect of the present invention. The composition may also include an adjuvant.

The vectors can be utilised with immunostimulatory agents. Preferably the immunostimulatory agent are administered at the same time as the nucleic acid vector of the invention and in preferred embodiments are formulated together. Such immunostimulatory agents include, but this list is by no means exhaustive and does not preclude other agents: synthetic imidazoquinolines such as imiquimod [S-26308, R-837], (Harrison, et al. 'Reduction of recurrent HSV disease using imiquimod alone or combined with a glycoprotein vaccine', Vaccine 19: 1820-1826, (2001)); and resiquimod [S-28463, R-848] (Vasilakos, et al. Adjuvant activites of immune response modifier R-848: Comparison with CpG ODN', Cellular immunology 204: 64-74 (2000).), Schiff bases of carbonyls and amines that are constitutively expressed on antigen presenting cell and T-cell surfaces, such as tucaresol (Rhodes, J. et al. Therapeutic potentiation of the immune system by costimulatory Schiff-base-forming drugs', Nature 377: 71-75 (1995)), cytokine, chemokine and co-stimulatory molecules as either protein or peptide, this would include pro-inflammatory cytokines such as GM-CSF, IL-1 alpha, IL-1 beta, TGF- alpha and TGF - beta, Th1 inducers such as interferon gamma, IL-2, IL-12, IL-15 and IL-18, Th2 inducers such as IL-4, IL-5, IL-6, IL-10 and IL-13 and other chemokine and co-stimulatory genes such as MCP-1, MIP-1 alpha, MIP-1 beta, RANTES, TCA-3, CD80, CD86 and CD40L, , other immunostimulatory targeting ligands such as CTLA-4 and L-selectin, apoptosis stimulating proteins and peptides such as Fas, (49), synthetic lipid based adjuvants, such as vaxfectin, (Reyes et al., 'Vaxfectin enhances antigen specific antibody titres and maintains Th1 type immune responses to plasmid DNA immunization', Vaccine 19: 3778-3786) squalene, alpha- tocopherol, polysorbate 80, DOPC and cholesterol, endotoxin, [LPS], Beutler, B., 'Endotoxin, 'Toll-like receptor 4, and the afferent limb of innate immunity', Current Opinion in Microbiology 3: 23-30 (2000)) ; CpG oligo- and di-nucleotides, Sato, Y. et al., 'Immunostimulatory DNA sequences necessary for effective intradermal gene immunization', Science 273 (5273): 352-354 (1996). Hemmi, H. et al., 'A Toll-like receptor recognizes bacterial DNA', Nature 408: 740-745, (2000) and other potential ligands that trigger Toll receptors to produce Th1-inducing cytokines, such as synthetic Mycobacterial lipoproteins, Mycobacterial protein p19, peptidoglycan, teichoic acid and lipid A.

Certain preferred adjuvants for eliciting a predominantly Th1-type response include, for example, a Lipid A derivative such as monophosphoryl lipid A, or preferably 3-de-O-acylated monophosphoryl lipid A. MPL^{®} adjuvants are available from Corixa Corporation (Seattle, WA; *see,* for example, US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462. Immunostimulatory DNA sequences are also described, for example, by Sato et al., Science 273:352, 1996. Another preferred adjuvant comprises a saponin, such as Quil A, or derivatives thereof, including QS21 and QS7 (Aquila Biopharmaceuticals Inc., Framingham, MA); Escin; Digitonin; or *Gypsophila* or *Chenopodium quinoa* saponins.

Also provided are the use of a polynucleotide according to the invention, or of a vector according to the invention, in the treatment or prophylaxis of an HIV infection.

The present invention also provides methods of treating or preventing HIV infections, any symptoms or diseases associated therewith, comprising administering an effective amount of a polynucleotide, a vector or a pharmaceutical composition according to the invention. Administration of a pharmaceutical composition may take the form of one or more individual doses, for example as repeat doses of the same DNA plasmid, or in a "prime-boost" therapeutic vaccination regime. In certain cases the "prime" vaccination may be via particle mediated DNA delivery of a polynucleotide according to the present invention, preferably incorporated into a plasmid-derived vector and the "boost" by administration of a recombinant viral vector comprising the same polynucleotide sequence, or boosting with the protein in adjuvant. Conversly the priming may be with the viral vector or with a protein formulation typically a protein formulated in adjuvant and the boost a DNA vaccine of the present invention. Multiple doses of prime and/or boost may be employed.

In embodiments of the invention fragments of gag, nef or RT proteins are contemplated. For example, a polynucleotide of the invention may encode a fragment of an HIV gag, nef or RT protein. A polynucleotide which encodes a fragment of at least 8, for example 8-10 amino acids or up to 20, 50, 60, 70, 80, 100, 150 or 200 amino acids in length is considered to fall within the scope of the invention as long as the encoded oligo or polypeptide demonstrates HIV antigenicity. In particular, but not exclusively, this aspect of the invention encompasses the situation when the polynucleotide encodes a fragment of a complete HIV protein sequence and may represent one or more discrete epitopes of that protein. Such fragments may be codon optimised such that the fragment has a codon usage pattern which resembles that of a highly expressed mammalian gene.

Disclosed constructs are:
1. p17, p24, fused to truncated NEF (devoid of nucleotides encoding terminal amino-acids 1-65)
2. p17, p24, RT, truncated NEF (devoid of nucleotides encoding terminal amino-acids 1-65)
3. p17, p24 (optimised gag) truncated NEF (devoid of nucleotides encoding terminal amino-acids 1-65)
4. p17, p24 (optimised gag) RT (optimised) truncated NEF (devoid of nucleotides encoding terminal amino-acids 1-85)
5. p17, p24, RT (optimised) truncated NEF (devoid of nucleotides encoding terminal amino-acids 1-65)
6. Truncated NEF - (devoid of nucleotide 1-65) fused to optimised p17, p24 gag.
7. Particularly preferred constructs of the invention include triple fusions RT-NEF-Gag, and RT-Gag-Nef particularly:
8. Optimised RT, truncated NEF and optimised P17, p24 (gag) (RNG)
   and
9. Optimised RT, optimised p17, 24 (gag), Nef truncate (devoid of aa 1-65)RGN

It is preferred that the HIV constructs are derived from an HIV Clade B or Clade C, particularly clade B.

As discussed above, the present invention includes expression vectors that comprise the nucleotide sequences of the invention. Such expression vectors are routinely constructed in the art of molecular biology and may for example involve the use of plasmid DNA and appropriate initiators, promoters, enhancers and other elements, such as for example polyadenylation signals which may be necessary, and which are positioned in the correct orientation, in order to allow for protein expression. Other suitable vectors would be apparent to persons skilled in the art. By way of further example in this regard we refer to Sambrook et al. Molecular Cloning: a Laboratory Manual. 2nd Edition. CSH Laboratory Press. (1989).

Preferably, a polynucleotide of the invention, or for use in the invention in a vector, is operably linked to a control sequence which is capable of providing for the expression of the coding sequence by the host cell, i.e. the vector is an expression vector. The term "operably linked" refers to a juxtaposition wherein the components described are in a relationship permitting them to function in their intended manner. A regulatory sequence, such as a promoter, "operably linked" to a coding sequence is positioned in such a way that expression of the coding sequence is achieved under conditions compatible with the regulatory sequence.

The vectors may be, for example, plasmids, artificial chromosomes (e.g. BAC, PAC, YAC), virus or phage vectors provided with a origin of replication, optionally a promoter for the expression of the polynucleotide and optionally a regulator of the promoter. The vectors may contain one or more selectable marker genes, for example an ampicillin or kanamycin resistance gene in the case of a bacterial plasmid or a resistance gene for a fungal vector. Vectors may be used in vitro, for example for the production of DNA or RNA or used to transfect or transform a host cell, for example, a mammalian host cell e.g. for the production of protein encoded by the vector. The vectors may also be adapted to be used in vivo, for example in a method of DNA vaccination or of gene therapy.

Promoters and other expression regulation signals may be selected to be compatible with the host cell for which expression is designed. For example, mammalian promoters include the metallothionein promoter, which can be induced in response to heavy metals such as cadmium, and the β-actin promoter. Viral promoters such as the SV40 large T antigen promoter, human cytomegalovirus (CMV) immediate early (IE) promoter, rous sarcoma virus LTR promoter, adenovirus promoter, or a HPV promoter, particularly the HPV upstream regulatory region (URR) may also be used. All these promoters are well described and readily available in the art.

A preferred promoter element is the CMV immediate early promoter, devoid of intron A but including exon 1. The promoter element may be the minimal promoter element or the enhanced promoter, the enhanced promoter being preferred. Accordingly there is provided a vector comprising a polynucleotide of the invention under the control of HCMV IE early promoter.

Examples of suitable viral vectors include herpes simplex viral vectors, vaccinia or alpha-virus vectors and retroviruses, including lentiviruses, adenoviruses and adeno-associated viruses. Gene transfer techniques using these viruses are known to those skilled in the art. Retrovirus vectors for example may be used to stably integrate the polynucleotide of the invention into the host genome, although such recombination is not preferred. Replication-defective adenovirus vectors by contrast remain episomal and therefore allow transient expression. Vectors capable of driving expression in insect cells (for example baculovirus vectors), in human cells, in yeast or in bacteria may be employed in order to produce quantities of the HIV protein encoded by the polynucleotides of the present invention, for example for use as subunit vaccines or in immunoassays.

The polynucleotides according to the invention have utility in the production by expression of the encoded proteins, which expression may take place *in vitro, in vivo* or *ex vivo.* The nucleotides may therefore be involved in recombinant protein synthesis, for example to increase yields, or indeed may find use as therapeutic agents in their own right, utilised in DNA vaccination techniques. Where the polynucleotides of the present invention are used in the production of the encoded proteins in vitro or *ex vivo,* cells, for example in cell culture, will be modified to include the polynucleotide to be expressed. Such cells include transient, or preferably stable mammalian cell lines. Particular examples of cells which may be modified by insertion of vectors encoding for a polypeptide according to the invention include mammalian HEK293T, CHO, HeLa, 293 and COS cells. Preferably the cell line selected will be one which is not only stable, but also allows for mature glycosylation and cell surface expression of a polypeptide. Expression may be achieved in transformed oocytes. A polypeptide may be expressed from a polynucleotide of the present invention, in cells of a transgenic non-human animal, preferably a mouse. A transgenic non-human animal expressing a polypeptide from a polynucleotide of the invention is included within the scope of the invention.

Disclosed is a method of vaccinating a mammalian subject which comprises administering thereto an effective amount of such a vaccine or vaccine composition. Most preferably, expression vectors for use in DNA vaccines, vaccine compositions and immunotherapeutics will be plasmid vectors.

DNA vaccines may be administered.in the form of "naked DNA", for example in a liquid formulation administered using a syringe or high pressure jet, or DNA formulated with liposomes or an irritant transfection enhancer, or by particle mediated DNA delivery (PMDD). All of these delivery systems are well known in the art. The vector may be introduced to a mammal for example by means of a viral vector delivery system.

The compositions of the present invention can be delivered intramuscularly.

In a preferred embodiment, the composition is delivered intradermally. In particular, the composition is delivered by means of a gene gun particularly particle bombardment administration techniques which involve coating the vector on to a bead (eg gold) which are then administered under high pressure into the epidermis; such as, for example, as described in Haynes et al, J Biotechnology 44: 37-42 (1996).

In one illustrative example, gas-driven particle acceleration can be achieved with devices such as those manufactured by Powderject Pharmaceuticals PLC (Oxford, UK) and Powderject Vaccines Inc. (Madison, WI), some examples of which are described in U.S. Patent Nos. 5,846,796; 6,010,478; 5,865,796; 5,584,807; and EP Patent No. 0500 799. This approach offers a needle-free delivery approach wherein a dry powder formulation of microscopic particles, such as polynucleotide, are accelerated to high speed within a helium gas jet generated by a hand held device, propelling the particles into a target tissue of interest, typically the skin.

The particles are preferably gold beads of a 0.4 - 4.0 µm, more preferably 0.6 - 2.0 µm diameter and the DNA conjugate coated onto these and then encased in a cartridge or cassette for placing into the "gene gun".

In a related embodiment, other devices and methods that may be useful for gas-driven needle-less injection of compositions of the present invention include those provided by Bioject, Inc. (Portland, OR), some examples of which are described in U.S. Patent Nos. 4,790,824; 5,064,413; 5,312,335; 5,383,851; 5,399,163; 5,520,639 and 5,993,412.

The vectors which comprise the nucleotide sequences encoding antigenic peptides are administered in such amount as will be prophylactically or therapeutically effective. The quantity to be administered, is generally in the range of one picogram to 1 milligram, preferably 1 picogram to 10 micrograms for particle-mediated delivery, and 100 nanograms to 1 milligram, preferably 10 micrograms to 1 milligram, for other routes, of nucleotide per dose. The exact quantity may vary considerably depending on the weight of the patient being immunised and the route of administration,

It is possible for the immunogen component comprising the nucleotide sequence encoding the antigenic peptide, to be administered on a once off basis or to be administered repeatedly, for example, between 1 and 7 times, preferably between 1 and 4 times, at intervals between about 1 day and about 18 months. However, this treatment regime will be significantly varied depending upon the size the patient concerned, the amount of nucleotide sequence administered, the route of administration, and other factors which would be apparent to a skilled veterinary or medical practitioner. The patient may receive one or more other anti HIV retroviral drugs as part of their overall treatment regime. Additionally the nucleic acid immunogen may be administered with an adjuvant.

The adjuvant component specified herein can similarly be administered via a variety of different administration routes, such as for example, via the oral, nasal, pulmonary, intramuscular, subcutaneous, intradermal or topical routes. Preferably, the adjuvant component is administered via the intradermal or topical routes. Most preferably by the topical route. This administration may take place between about 14 days prior to and about 14 days post administration of the nucleotide sequence, preferably between about 1day prior to and about 3 days post administration of the nucleotide sequence. The adjuvant component is, in an embodiment, administered substantially simultaneously with the administration of the nucleotide sequence. By "substantially simultaneous" what is meant is that administration of the adjuvant component is preferably at the same time as administration of the nucleotide sequence, or if not, at least within a few hours either side of nucleotide sequence administration. In the most preferred treatment protocol, the adjuvant component will be administered substantially simultaneously to administration of the nucleotide sequence. Obviously, this protocol can be varied as necessary, in accordance with the type of variables referred to above. It is preferred that the adjuvant is a 1H - imidazo [4,5c] quinoline - 4 - amine derivative such as imiquimod. Typically imiquimod will be presented as a topical cream formulation and will be administered according to the above protocol.

Once again, depending upon such variables, the dose of administration of the derivative will also vary, but may, for example, range between about 0.1mg per kg to about 100mg per kg, where "per kg" refers to the body weight of the mammal concerned. This administration of the, 1H-imidazo[4,5-c]quinolin-4-amine derivative would preferably be repeated with each subsequent or booster administration of the nucleotide sequence. Most preferably, the administration dose will be between about 1mg per kg to about 50mg per kg. In the case of a "prim-boost" scheme as described herein, the imiquimod or other 1H-imidazo[4,5-c]quinolin-4-amine derivative may be administered with either the prime or the boost or with both the prime and the boost.

While it is possible for the adjuvant component to comprise only 1H-imidazo[4,5-c]quinolin-4-amine derivatives to be administered in the raw chemical state, it is preferable for administration to be in the form of a pharmaceutical formulation. That is, the adjuvant component will preferably comprise the 1H-imidazo[4,5-c]quinolin-4-amine combined with one or more pharmaceutically acceptable carriers, and optionally other therapeutic ingredients. The carrier(s) must be "acceptable" in the sense of being compatible with other ingredients within the formulation, and not deleterious to the recipient thereof. The nature of the formulations will naturally vary according to the intended administration route, and may be prepared by methods well known in the pharmaceutical art. All methods include the step of bringing into association a 1H-imidazo[4,5-c]quinolin-4-amine derivative with an appropriate carrier or carriers. In general, the formulations are prepared by uniformly and intimately bringing into association the derivative with liquid carriers or finely divided solid carriers, or both, and then, if necessary, shaping the product into the desired formulation. Formulations of the present invention suitable for oral administration may be presented as discrete units such as capsules, cachets or tablets each containing a pre-determined amount of the active ingredient; as a powder or granules; as a solution or a suspension in an aqueous liquid or a non-aqueous liquid; or as an oil-in-water liquid emulsion or a water-in-oil emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent.

The tablets may optionally be coated or scored and may be formulated so as to provide slow or controlled release of the active ingredient.

Formulations for injection via, for example, the intramuscular, intraperitoneal, or subcutaneous administration routes include aqueous and non-aqueous sterile injection solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampoules and vials, and may be stored in a freeze-dried (lyophilised) condition requiring only the addition of the sterile liquid carrier, for example, water for injections, immediately prior to use. Extemporaneous injection solutions and suspensions may be prepared from sterile powders, granules and tablets of the kind previously described. Formulations suitable for pulmonary administration via the buccal or nasal cavity are presented such that particles containing the active ingredient, desirably having a diameter in the range of 0.5 to 7 microns, are delivered into the bronchial tree of the recipient. Possibilities for such formulations are that they are in the form of finely comminuted powders which may conveniently be presented either in a piercable capsule, suitably of, for example, gelatine, for use in an inhalation device, or alternatively, as a self-propelling formulation comprising active ingredient, a suitable liquid propellant and optionally, other ingredients such as surfactant and/or a solid diluent. Self-propelling formulations may also be employed wherein the active ingredient is dispensed in the form of droplets of a solution or suspension. Such self-propelling formulations are analogous to those known in the art and may be prepared by established procedures. They are suitably provided with either a manually-operable or automatically functioning valve having the desired spray characteristics; advantageously the valve is of a metered type delivering a fixed volume, for example, 50 to 100 µL, upon each operation thereof.

In a further possibility, the adjuvant component may be in the form of a solution for use in an atomiser or nebuliser whereby an accelerated airstream or ultrasonic agitation is employed to produce a find droplet mist for inhalation.

Formulations suitable for intranasal administration generally include presentations similar to those described above for pulmonary administration, although it is preferred for such formulations to have a particle diameter in the range of about 10 to about 200 microns, to enable retention within the nasal cavity. This may be achieved by, as appropriate, use of a powder of a suitable particle size, or choice of an appropriate valve. Other suitable formulations include coarse powders having a particle diameter in the range of about 20 to about 500 microns, for administration by rapid inhalation through the nasal passage from a container held close up to the nose, and nasal drops comprising about 0.2 to 5% w/w of the active ingredient in aqueous or oily solutions. In one embodiment of the invention, it is possible for the vector which comprises the nucleotide sequence encoding the antigenic peptide to be administered within the same formulation as the 1H-imidazo[4,5-c]quinolin-4-amine derivative. Hence in this embodiment, the immunogenic and the adjuvant component are found within the same formulation.

In an embodiment the adjuvant component is prepared in a form suitable for gene-gun administration, and is administered via that route substantially simultaneous to administration of the nucleotide sequence. For preparation of formulations suitable for use in this manner, it may be necessary for the 1H-imidazo[4,5-c]quinolin-4-amine derivative to be lyophilised and adhered onto, for example, gold beads which are suited for gene-gun administration.

In an alternative embodiment, the adjuvant component may be administered as a dry powder, via high pressure gas propulsion.

Even if not formulated together, it may be appropriate for the adjuvant component to be administered at or about the same administration site as the nucleotide sequence.

Other details of pharmaceutical preparations can be found in Remington's Pharmaceutical Sciences, Mack Publishing Company, Easton, Pennysylvania (1985), the disclosure of which is included herein in its entirety, by way of reference.

Suitable techniques for introducing the naked polynucleotide or vector into a patient also include topical application with an appropriate vehicle. The nucleic acid may be administered topically to the skin, or to mucosal surfaces for example by intranasal, oral, intravaginal or intrarectal administration. The naked polynucleotide or vector may be present together with a pharmaceutically acceptable excipient, such as phosphate buffered saline (PBS). DNA uptake may be further facilitated by use of facilitating agents such as bupivacaine, either separately or included in the DNA formulation. Other methods of administering the nucleic acid directly to a recipient include ultrasound, electrical stimulation, electroporation and microseeding which is described in US-5,697,901.

Uptake of nucleic acid constructs may be enhanced by several known transfection techniques, for example those including the use of transfection agents. Examples of these agents includes cationic agents, for example, calcium phosphate and DEAE-Dextran and lipofectants, for example, lipofectam and transfectam. The dosage of the nucleic acid to be administered can be altered.

A nucleic acid sequence may also be administered by means of specialised delivery vectors useful in gene therapy. Gene therapy approaches are discussed for example by Verme et al, Nature 1997, 389:239-242. Both viral and non-viral vector systems can be used. Viral based systems include retroviral, lentiviral, adenoviral, adeno-associated viral, herpes viral, Canarypox and vaccinia-viral based systems. Non-viral based systems include direct administration of nucleic acids, microsphere encapsulation technology (poly(lactide-co-glycolide) and, liposome-based systems. Viral and non-viral delivery systems may be combined where it is desirable to provide booster injections after an initial vaccination, for example an initial "prime" DNA vaccination using a non-viral vector such as a plasmid followed by one or more "boost" vaccinations using a viral vector or non-viral based system. Similarly the invention contemplates prime boot systems with the polynucleotide of the invention, followed by boosting with protein in adjuvant or vice versa.

A nucleic acid sequence may also be administered by means of transformed cells. Such cells include cells harvested from a subject. The naked polynucleotide or vector of the present invention can be introduced into such cells in vitro and the transformed cells can later be returned to the subject. The polynucleotide of the invention may integrate into nucleic acid already present in a cell by homologous recombination events. A transformed cell may, if desired, be grown up in vitro and one or more of the resultant cells may be used in the present invention. Cells can be provided at an appropriate site in a patient by known surgical or microsurgical techniques (e.g. grafting, micro-injection, etc.)

The pharmaceutical compositions may include adjuvant compounds, as detailed above, or other substances which may serve to increase the immune response induced by the protein which is encoded by the DNA. These may be encoded by the DNA, either separately from or as a fusion with the antigen, or may be included as non-DNA elements of the formulation. Examples of adjuvant-type substances which may be included in the formulations of the present invention include ubiquitin, lysosomal associated membrane protein (LAMP), hepatitis B virus core antigen, FLT3-ligand (a cytokine important in the generation of professional antigen presenting cells, particularly dentritic cells) and other cytokines such as IFN-γ and GMCSF. Other preferred adjuvants include Imiquimod and Resimquimod and Tucarasol. Imiquimod being particularly preferred.

The present invention in a preferred embodiments of the invention provides a nucleic acid molecule as herein described for use in treatment or prophylaxis of HIV infection. The nucleic acid molecule is preferably administered with Imiquimod. The Imiquimod is preferably administered topically, whereas the nucleic acid molecule is preferably administered by means of the particle mediated delivery.

Accordingly a method of treating a subject suffering from or susceptible to HIV infection, comprising administering a nucleic acid molecule as herein described and Imiquimod is provided.

The present invention will now be described by reference to the following examples:

### EXAMPLES

### Example 1: Optimisation of p55 gag (p17, p24, p13) to resemble codon usage of highly expressed human genes.

### Gene of interest

A synthetic gene coding for the p55gag antigen of the HIV-1 clade B strain HXB2 (GenBank entry K03455), optimised for expression in mammalian cells was assembled from overlapping oligonucleotides by PCR.

Optimisation involved changing the codon usage pattern of the viral gene to give a codon frequency closer to that found in highly expressed human genes. Codons were assigned using a statistical Visual Basic program called Syngene (an updated version of Calcgene, written by R.S. Hale and G. Thompson, Protein Expression and Purification Vol. 12 pp 185-188, 1998)

### Cloning:

The 1528bp gag PCR product was gel purified, cut with restriction endonucleases NotI and Bam HI and ligated into NotI/BamHI cut vector WRG7077. This places the gene between the CMV promoter/intron A and the Bovine growth hormone polyadenylation signal.

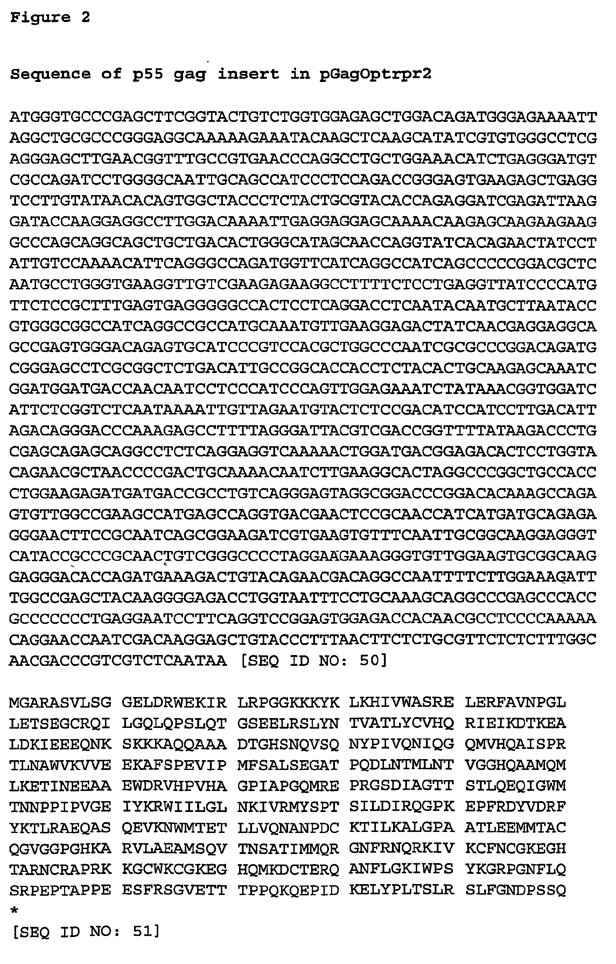

Clones were sequenced and checked for errors. No single clone was 100% correct. Regions of correct sequence from two clones were therefore combined by overlapping PCR using appropriate combinations of the optimisation oligo set to give a full length codon optimised gag gene. This final clone was subsequently found to contain a single nucleotide deletion which resulted in a frame shift and premature termination of translation. The deletion was repaired by cutting out the region of the gene containing the incorrect sequence and cloning in the correct sequence from the equivalent region of another clone. This gave the final codon optimised p55 gag clone: Gagoptrpr2. (See figure 2)

### Example 2: Production of a p17/p24 truncated Nef fusion gene

### Gene of interest

The p17 and p24 portions of the p55gag gene derived from the HIV-1 clade B strain HXB2 was PCR amplified from the plasmid pHXB?Pr (B.Maschera, E Furfine and E.D. Blair 1995 J.Virol 69 5431-5436). pHXB?Pr. 426bp from the 3' end of the HXB2 nef gene were amplified from the same plasmid. Since the HXB2 nef gene contains a premature termination codon two overlapping PCRs were used to repair the codon (TGA [stop] to TGG [Trp])

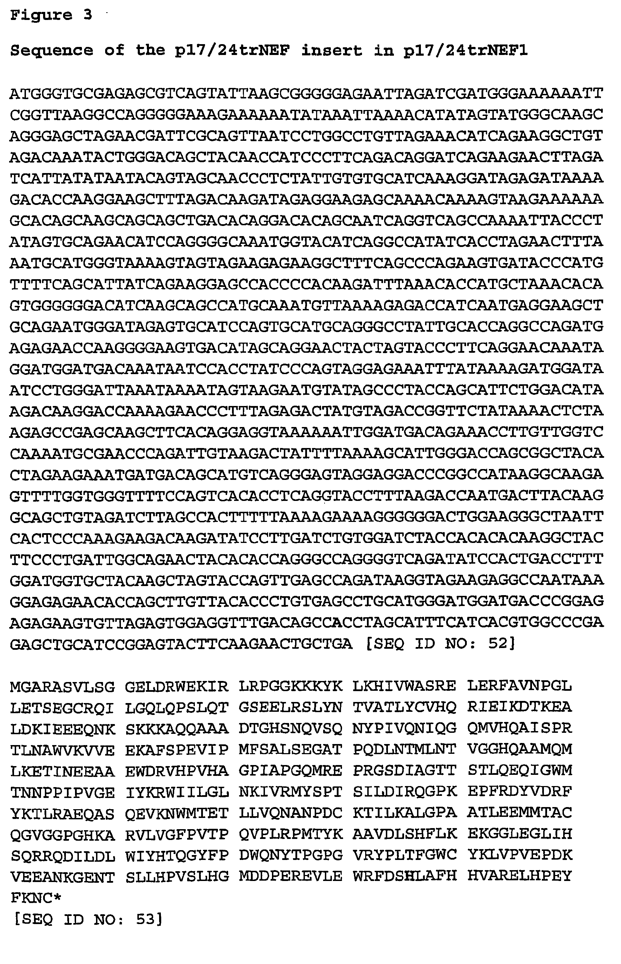

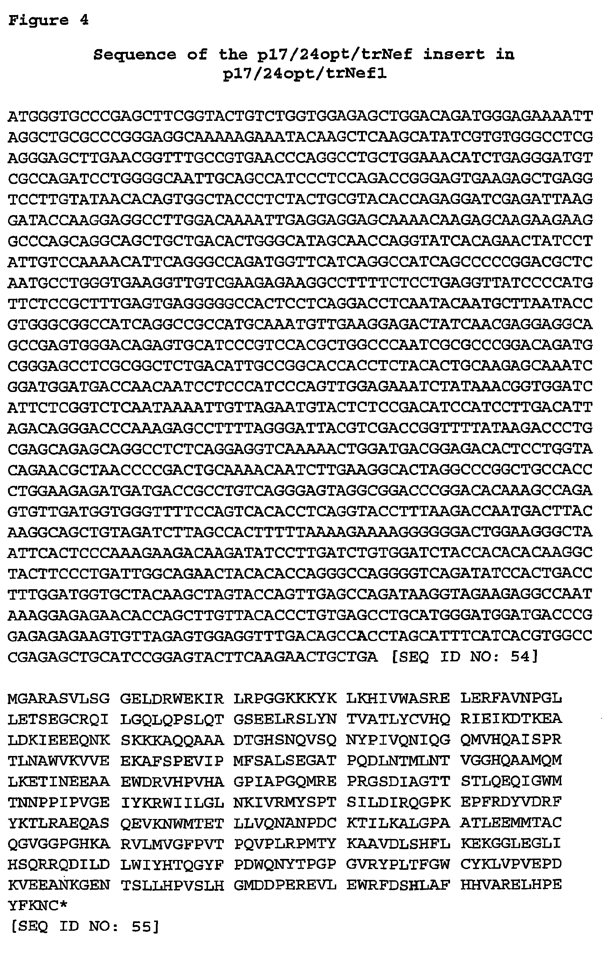

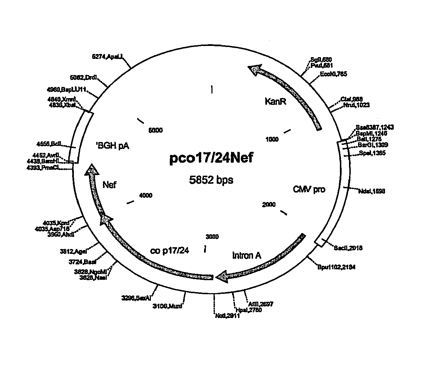

The p17/p24linker and trNEFlinker PCR products were joined to form the p17p24trNEF fusion gene (figure 3) in a PCR reaction **(antisense)**

The 1542bp product was gel purified, cut with restriction endonucleases NotI and BamHI and cloned into the NotI BamHI sites of vector WRG7077. This places the gene between the CMV promoter/intron A and the Bovine growth hormone polyadenylation signal.

### Example 3: Production of an Gag p17/24opt/trNef1 ('Gagopt/Nef') fusion gene.

### Gene of interest

The p17/p24 portion of the codon optimised p55gag gene derived from the HIV-1 clade B strain HXB2 was PCR amplified from the plasmid pGagOPTrpr2. The truncated HXB2 Nef gene with the premature termination codon repaired (TGA [stop] to TGG [Trp]) was amplified by PCR from the plasmid 7077trNef20. The two PCR products were designed to have overlapping ends so that the two genes could be joined in a second PCR.

The 1544bp product was gel purified, cut with restriction endonucleases NotI and BamHI and cloned (see figures) into the NotI BamHI sites of vector WRG7077. This places the gene between the CMV promoter/intron A and the Bovine growth hormone polyadenylation signal.

### Example 4: Plasmid: p7077-RT3 Clone #A

### Gene of interest:

A synthetic gene coding for the RT portion of the pol gene of HIV-1 clade B strain HXB2, optimised for expression in mammalian cells assembled from overlapping oligonucleotides by PCR. The sequence cloned is equivalent to positions 2550-4222 of the HXB2 reference sequence (GenBank entry K03455). To ensure expression the cloned sequence has two additional codons at the 5' end not present in the original gene - AUG GGC (Met Gly). Optimisation involved changing the codon usage pattern of the viral gene to give a codon frequency closer to that found in highly expressed human genes, but excluding rarely used codons. Codons were assigned using a statistical Visual Basic program called Syngene (an updated version of Calcgene, written by R.S. Hale and G. Thompson, Protein Expression and Purification Vol. 12 pp 185-188, 1998)

The final clone was constructed from two intermediate clones, # 16 and #21.

### Cloning:

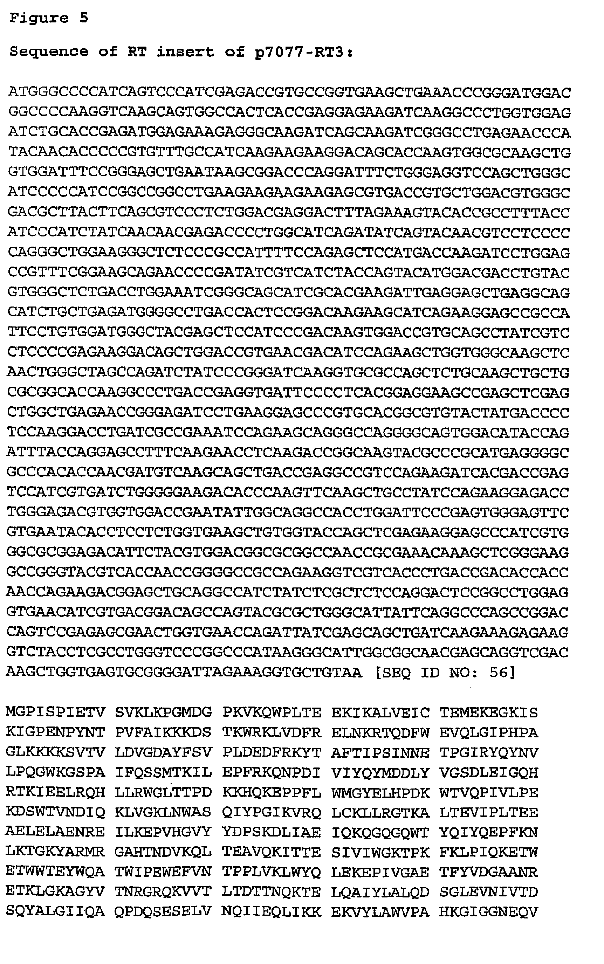

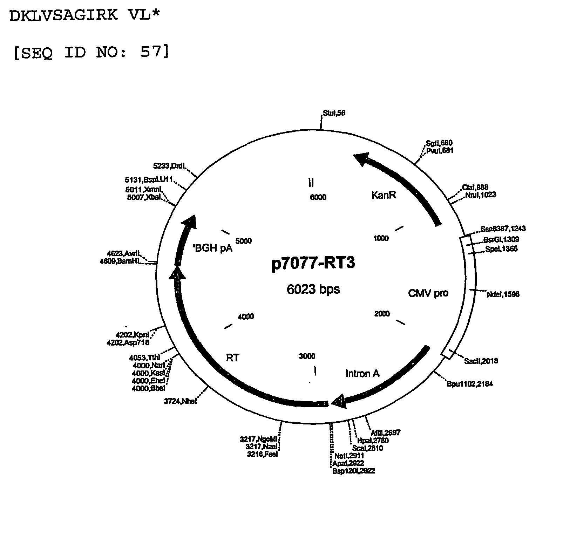

The 1.7kb PCR products were gel purified, cut with NotI and BamHI and PCR cleaned, before being ligated with NotI / BamHI cut pWRG7077. This places the gene between the CMV promoter and bovine growth hormone polyadenylation signal. Clones were sequenced. No clone was 100% correct, but clone #16 was corrected by replacing the 403bp KpnI-BamHI fragment containing 3 errors with a correct Kpn1-BamHI fragment from clone#21. The final clone was verified by sequencing. (see figure 5)

### Example 5: Optimised RT

### Gene of interest

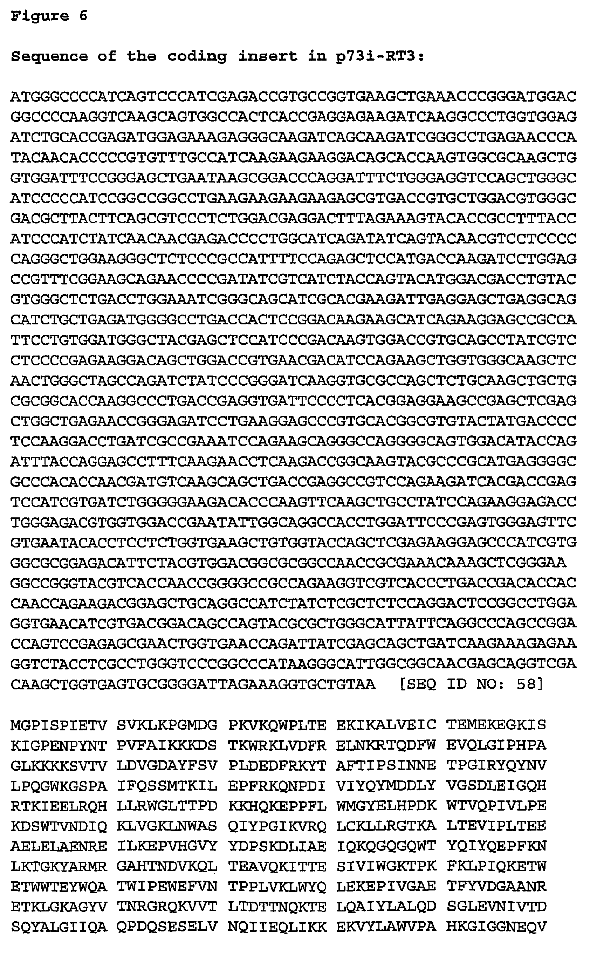

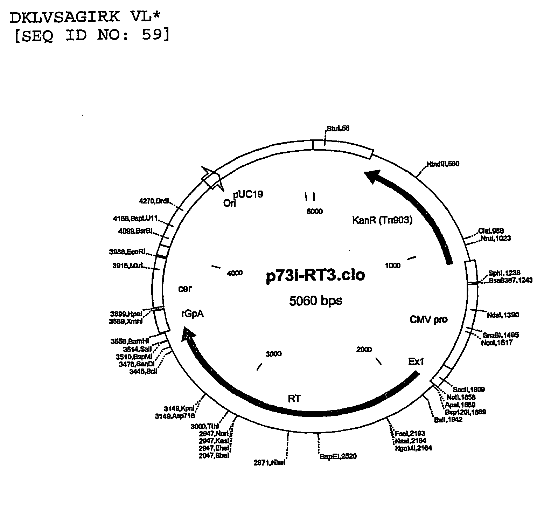

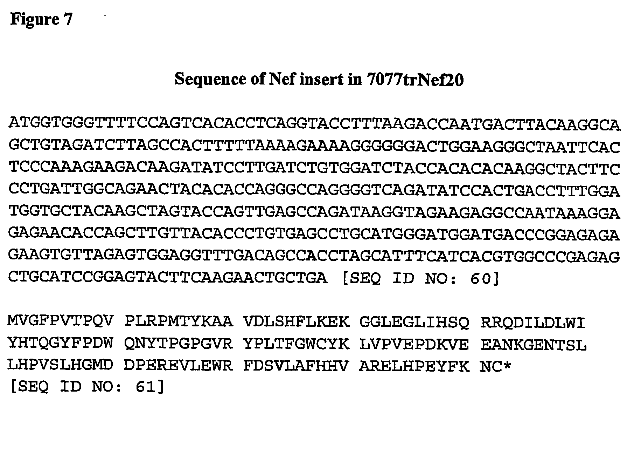

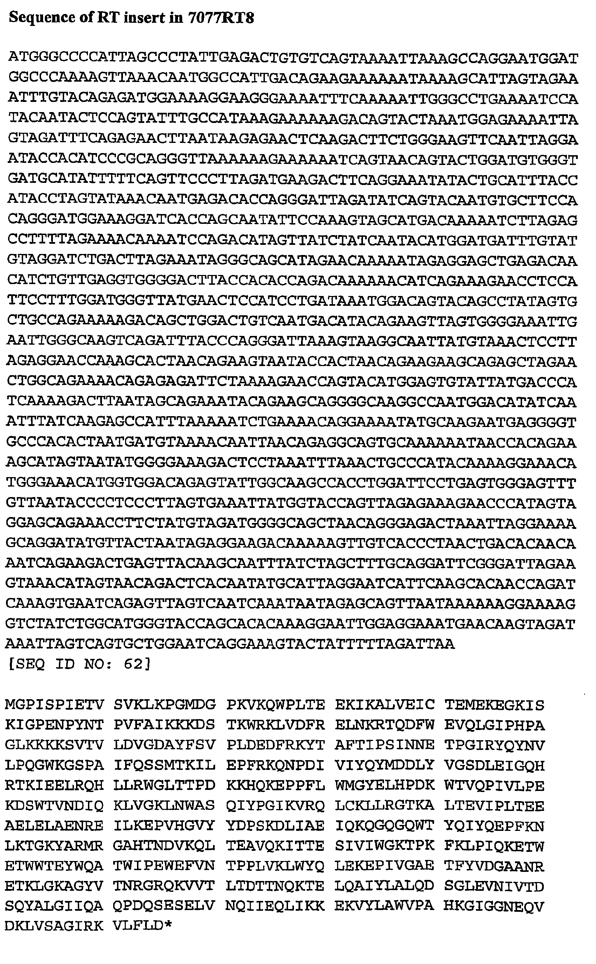

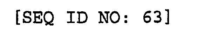

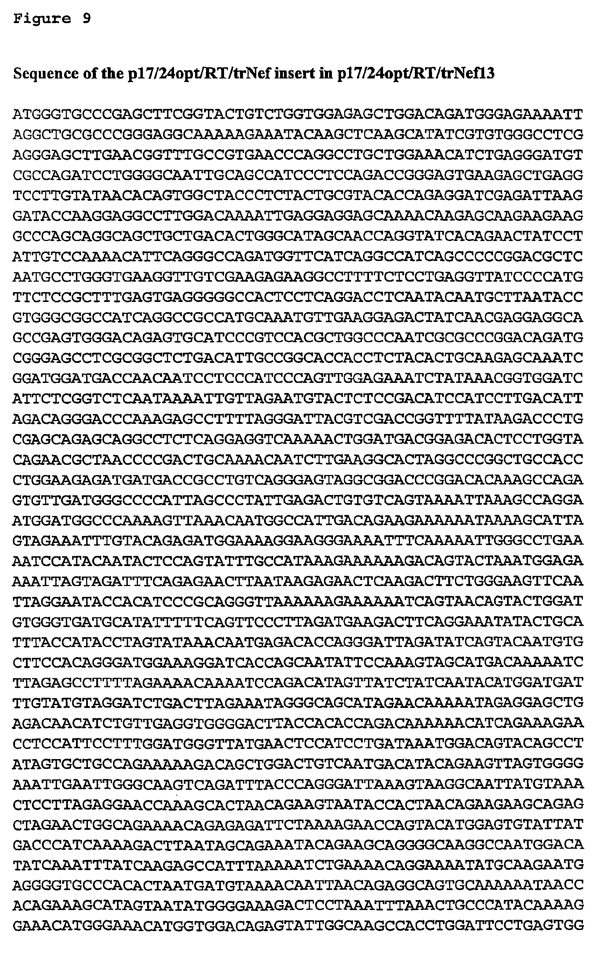

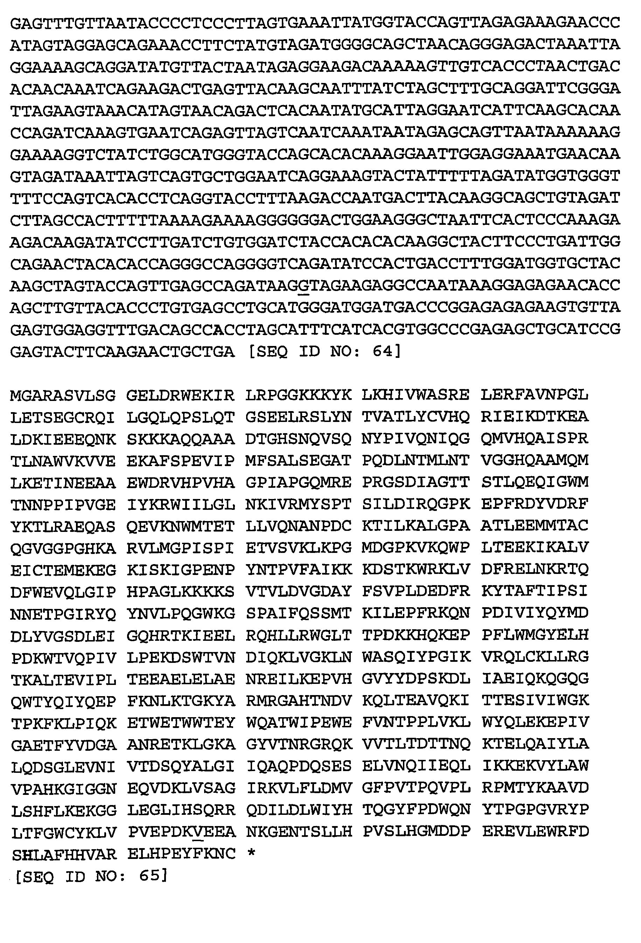

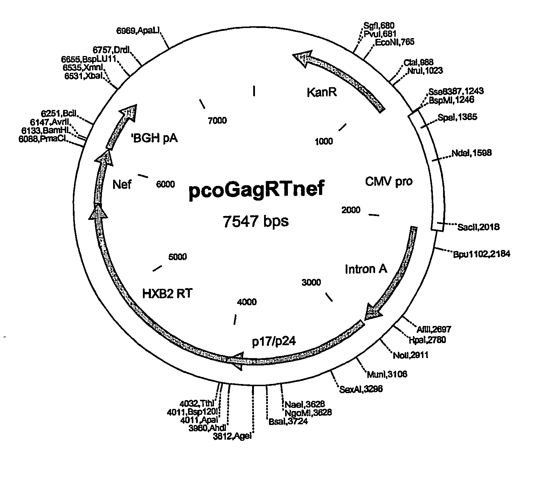

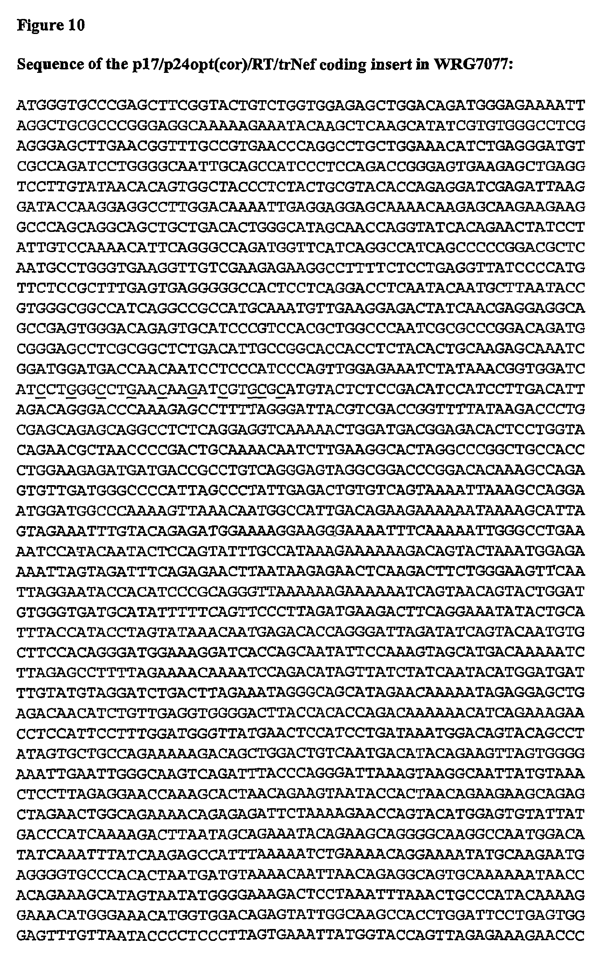

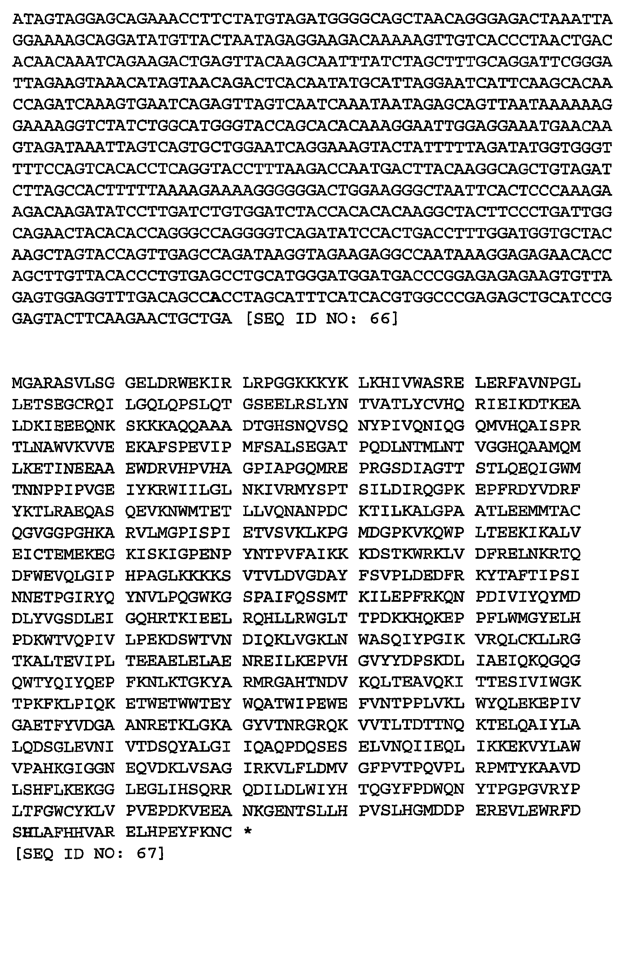

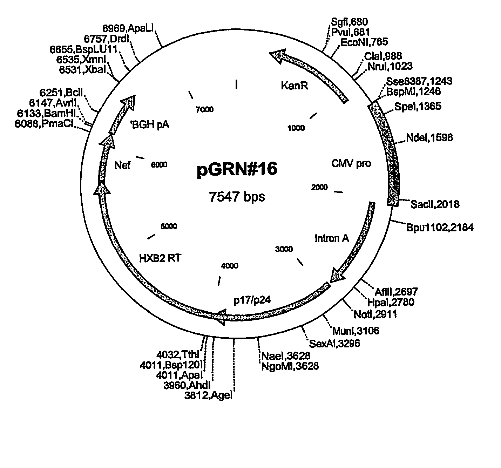

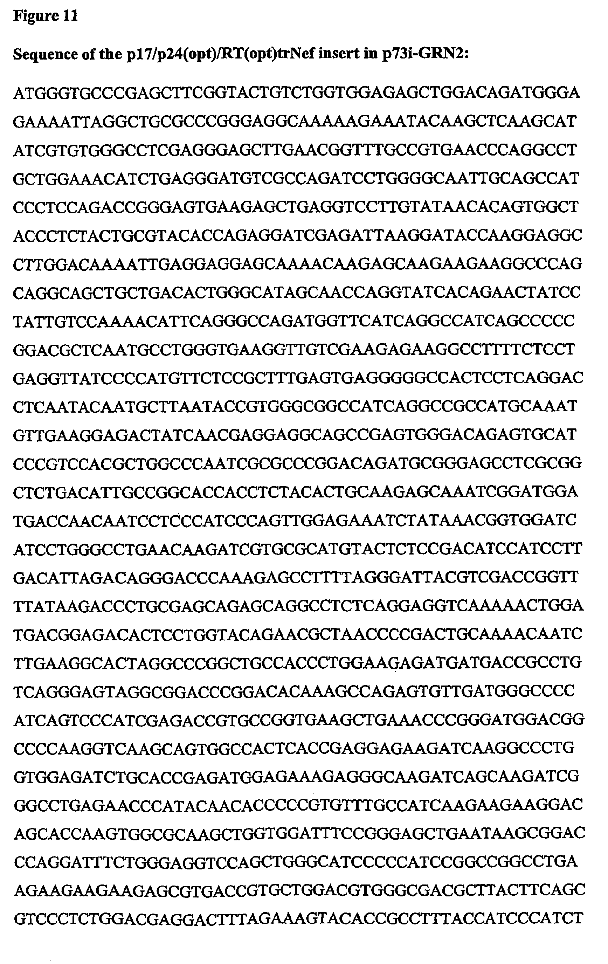

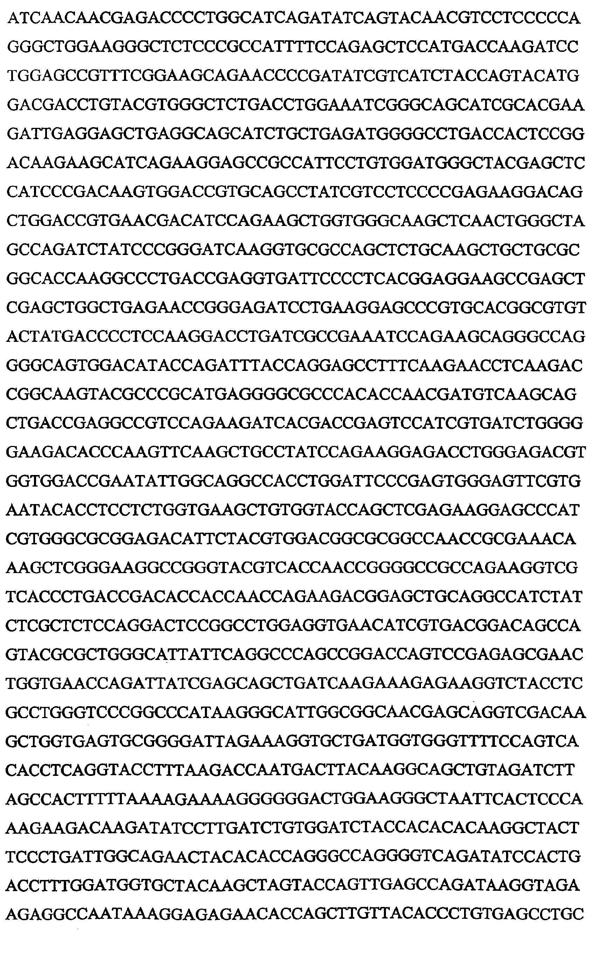

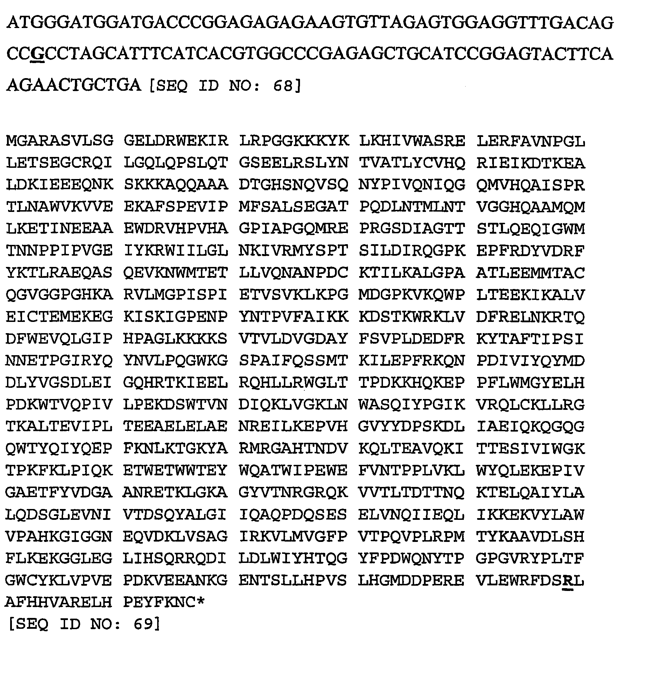

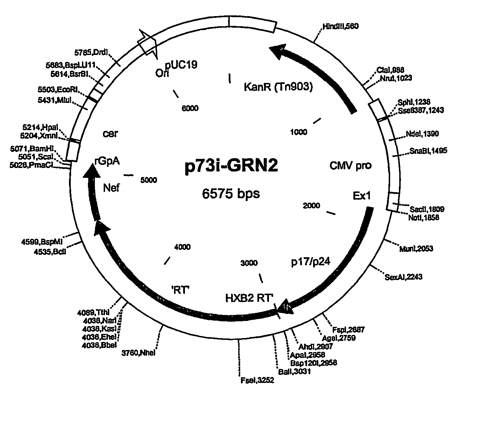

The synthetic gene coding for the RT portion of the pol gene of HIV-1 clade B strain HXB2, optimised for expression in mammalian cells was excised from plasmid p7077-RT3 as a 1697bp NotI / BamHI fragment, gel purified, and cloned into the NotI & BamHI sites of p7313-ie (derived from pspC31) to place the gene downstream of an Iowa length HCMV promoter + exon1, and upstream of a rabbit globin poly-adenylation signal. (R7004 p27) (figure 6)

### Example 6

### Plasmid: 7077trNef20

### Gene of interest

The insert comprises part of the Nef gene from the HIV-1 clade B strain HXB2. 195bp are deleted from the 5' end of the gene removing the codons for the first 65 amino acids of Nef. In addition the premature termination codon in the published HXB2 nef sequence has been repaired (TAG to TGG [Trp]) as has been described for plasmid p17/24trNEF1. The truncated nef sequence was PCR amplified from the plasmid p17/24trNef1. The sequence cloned is equivalent to positions 8992-9417 of the HXB2 reference sequence (GenBank entry K03455). To ensure expression the cloned sequence has an additional codon at the 5' end not present in the original gene - AUG (Met).

### Primers:

StrNef (sense) ATAAGAATGCGGCCGCCATGGTGGGTTTTCCAGTCACACCTT [SEQ ID NO: 1]
AStrNef (antisense)
   CGCGGATCCTCAGCAGTTCTTGAAGTACTCC [SEQ ID NO: 2]
PCR: 94° C 2min, then 25 cycles: 94° C 30sec, 50° C 30sec, 72° C 2min, ending 72° C 5min

### Cloning:

The 455bp RT PCR product was gel purified, cut with restriction endonucleases NotI and Bam HI and ligated into NotI/BamHI cut vector WRG7077. This places the gene between the CMV promoter/intron A and the Bovine growth hormone polyadenylation signal.

### Example 7

### Plasmid: 7077RT 8

### Gene of interest

The RT portion of the pol gene was derived from the HIV-1 clade B strain HXB2. It was PCR amplified from the plasmid p7077Pol14.

The sequence cloned is equivalent to positions 2550-4234 of the HXB2 reference sequence (GenBank entry K03455). To ensure expression the cloned sequence has two additional codons at the 5' end not present in the original gene - AUG GGC (Met Gly).

### Primers:

SRT (sense) ATAAGAATGCGGCCGCCATGGGCCCCATTAGCCCTATTGAGACT [SEQ ID NO: 3]
   ASRT (antisense)
   CGCGGATCCTTAATCTAAAAATAGTACTTTCCTGATT [SEQ ID NO: 4]
PCR: 94° C 2min, then 25 cycles: 94° C 30sec, 50° C 30sec, 72° C 4min, ending 72° C 5min

### Cloning:

The 1720bp RT PCR product was gel purified, cut with restriction endonucleases NotI and Bam HI and ligated into NotI/BamHI cut vector WRG7077. This places the gene between the CMV promoter/intron A and the Bovine growth hormone polyadenylation signal.

### Example 8

### p17/24opt/RT/trNef13 ('Gagopt/RT/Nef')

This construct contains a PCR that causes an R to H amino acid change.

### Gene of interest:

The p17/p24 portion of the codon optimised p55gag gene derived from the HIV-1 clade B strain HXB2 was PCR amplified from the plasmid pGagOPTrpr2. The RT coding sequence was PCR amplified from the plasmid 7077RT 8. The truncated HXB2 Nef gene with the premature termination codon repaired (TGA [stop] to TGG [Trp]) was amplified by PCR from the plasmid 7077trNef20. The three PCR products were designed to have overlapping ends so that the three genes could be joined in a second PCR.

### Primers:

### (P17/24)

Sp17p24opt (sense)
   ATAAGAATGCGGCCGCCATGGGTGCCCGAGCTTCGGT [SEQ ID NO: 5]
ASp17p24optRTlinker (antisense)
   TGGGGCCCATCAACACTCTGGCTTTGTGTC [SEQ ID NO: 6]
PCR: 94° C 1min, then 20 cycles: 94° C 30sec, 50° C 30sec, 72° C 2min, ending 72° C 4min

The 1114bp p17/24opt product was gel purified.

### (RT)

Sp17p24optRTlinker (sense)
   CAGAGTGTTGATGGGCCCCATTAGCCCTAT [SEQ ID NO: 7]
ASRTtrNeflinker (antisense)
   AACCCACCATATCTAAAAATAGTACTTTCC [SEQ ID NO: 8]
PCR: as above

The 1711bp RT PCR product was gel purified

### (5' truncated nef)

SRTtrNef linker (sense)
   CTATTTTTAGATATGGTGGGTTTTCCAGTCAC [SEQ ID NO: 9]
AStrNef (antisense)
   CGCGGATCCTCAGCAGTTCTTGAAGTACTCC [SEQ ID NO: 10]

PCR as above.

The 448bp product was gel purified.

The three PCR products were then stitched together in a second PCR with primers Sp17/24opt and AstrNef.
PCR: 94° C 1min, then 30 cycles: 94° C 30sec, 50° C 30sec, 72° C 4min, ending 72° C 4min

The 3253bp product was gel purified, cut with restriction endonucleases NotI and BamHI and cloned into the NotI BamHI sites of vector WRG7077. This places the gene between the CMV promoter/intron A and the Bovine growth hormone polyadenylation signal.

### Example 9

### Plasmid: pGRN#16 (p17/p24opt corr /RT/trNef.)

### Gene of interest:

The polyprotein generated by p17/24opt/RT/trNef13 ('Gagopt/RT/Nef') was observed to express a truncated product of ∼30kDa due to a cluster of unfavourable codons within p24 around aminoacid 270. These were replaced with optimal codons by PCR stitching mutagenesis. p17/24opt/RT/trNef13 was used as a template to amplify the portion of Gag 5' to the mutation with primers Sp17/p24opt and GTR-A, and the portion of Gag 3' to the mutation with primers GTR-S and Asp17/p24optRTlinker. The overlap of the products contained the codon changes, and the gel purified products were stitched together using the Sp17/p24o and Asp17/p24optRTlinker primers. The product was cut with NotI and AgeI and inserted into similarly cut p17/24opt/RT/trNef13, to generate pGRN. Clone #16 was verified and progressed.

### Primers:

### 5' PCR:

Sp17p24opt (sense)
   ATAAGAATGCGGCCGCCATGGGTGCCCGAGCTTCGGT [SEQ ID NO: 11]
GTR-A (Antisense)

GCGCACGATCTTGTTCAGGCCCAGGATGATCCACCGTTTATAGATTTCTCC [SEQ
ID NO: 12]

### 3' PCR

Sense: GTR-S(Sense) 
ATCCTGGGCCTGAACAAGATCGTGCGCATGTACTCTCCGACATCCATCC [SEQ ID
NO: 13]
ASp17p24optRTlinker (antisense)
   TGGGGCCCATCAACACTCTGGCTTTGTGTC [SEQ ID NO: 14]

PCR conditions for individual products and stitch, using PWO DNA polymerase (Roche):
95°C 1min, then 20 cycles 95°C 30s, 55°C 30s, 72°C 180s, ending 72°C 120s and 4°C hold.

The 1114bp product was gel purified and cut with NotI and AgeI to release a 6647bp fragment which was gel purified and ligated into NotI / AgeI cut gel purified p17/24opt/RT/trNef13 to generate pGRN#16.

### Example 10:

### Plasmid: p73i-GRN2 Clone #19

### (p17/p24(opt)/RT(opt)trNef) - repaired

### Gene of interest:

The p17/p24 portion of the codon optimised gag, codon optimised RT and truncated Nef gene from the HIV-1 clade B strain HXB2 downstream of an Iowa length HCMV promoter + exon1, and upstream of a rabbit β-globin poly-adenylation signal.

Plasmids containing the trNef gene derived from plasmid p17/24trNef1 contain a PCR error that gives an R to H amino acid change 19 amino acids from the end of nef. This was corrected by PCR mutagenesis, the corrected nef PCR stitched to codon optimised RT from p7077-RT3, and the stitched fragment cut with ApaI and BamHI, and cloned into ApaI/BamHI cut p73i-GRN.

### Primers:

PCR coRT from p7077-RT3 using primers:
   (Polymerase = PWO (Roche) throughout.

### Sense: U1

GAATTCGCGGCCGCGATGGGCCCCATCAGTCCCATCGAGACCGTGCCGGTGAAGCTG
AAACCCGGGAT [SEQ ID NO: 15]

AScoRT-Nef

GGTGTGACTGGAAAACCCACCATCAGCACCTTTCTAATCCCCGC [SEQ ID NO:
16]

Cycle: 95°C(30s) then 20 cycles 95°C(30s), 55°C(30s), 72°C(180s), then 72°C(120s) and hold at 4°C The 1.7kb PCR product was gel purified.

### PCR 5' Nef from p17/24trNef1 using primers:

Sense: S-Nef
   ATGGTGGGTTTTCCAGTCACACC [SEQ ID NO: 17]
Antisense: ASNef-G:
   GATGAAATGCTAGGCGGCTGTCAA.ACCTC [SEQ ID NO: 18]
Cycle: 95°C(30s) then 15 cycles 95°C(30s), 55°C(30s), 72°C(60s), then 72°C(120s) and hold at 4°C

### PCR 3' Nef from p17/24trNef1 using primers:

Sense: SNEF-G
   GAGGTTTGACAGCCGCCTAGCATTTCATC [SEQ ID NO: 19]
Antisense:
   AStrNef (antisense)
   CGCGGATCCTCAGCAGTTCTTGAAGTACTCC [SEQ ID NO: 20]
Cycle: 95°C(30s) then 15 cycles 95°C(30s), 55°C(30s), 72°C(60s), then 72°C(120s) and hold at 4°C

The PCR products were gel purified. Initially the two Nef products were stitched using the 5' (S-Nef) and 3' (AstrNef) primers.
Cycle: 95°C(30s) then 15 cycles 95°C(30s), 55°C(30s), 72°C(60s), then 72°C(180s) and hold at 4°C.
The PCR product was PCR cleaned, and stitched to the RT product using the U1 and AstrNef primers:
Cycle: 95°C(30s) then 20 cycles 95°C(30s), 55°C(30s), 72°C(180s), then 72°C(180s) and hold at 4°C

The 2.1kb product was gel purified, and cut with ApaI and BamHI. The plasmid p73I-GRN was also cut with Apa1 and BamHI gel purified and ligated with the ApaI-Bam RT3trNef to regenerate the p17/p24(opt)/RT(opt)trNef gene.

### Example 11

### p73i-GN2 Clone #2 (p17/p24opt/trNef) - repaired

### Gene of interest:

The p17/p24 portion of the codon optimised gag and truncated Nef genes from the HIV-1 clade B strain HXB2 downstream of an Iowa length HCMV promoter + exon1, and upstream of a rabbit β-globin poly-adenylation signal.

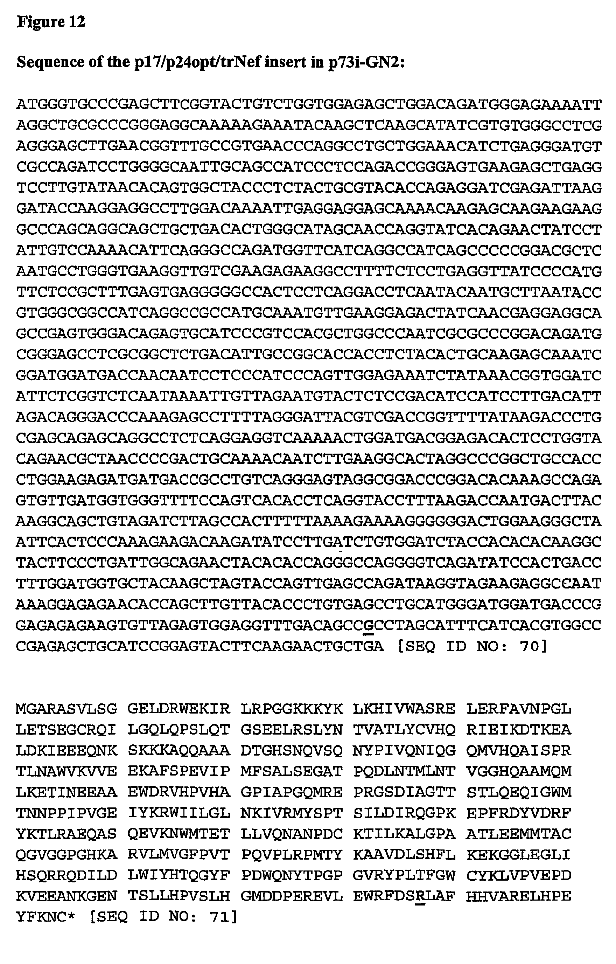

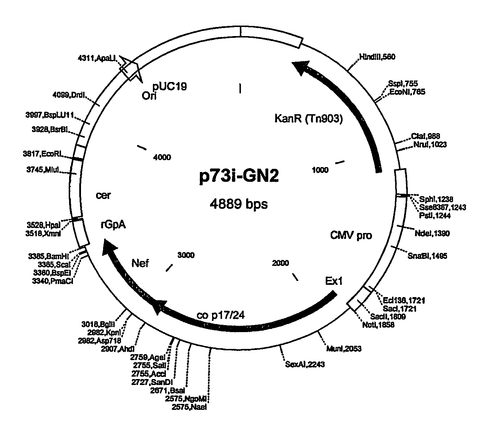

Plasmids containing the trNef gene derived from plasmid p17/24trNef1 contain a PCR error that gives an R to H amino acid change 19 amino acids from the end of Nef. This was corrected by PCR mutagenesis and the corrected fragment cut with BglII and BamHI, and cloned into BglII/BamHI cut p73I-GN. (Figure 12) regenerate the corrected p17/p24opt/trNef fusion gene downstream of the Iowa length HCMV promoter + exon1, and upstream of the rabbit β-globin polyadenylation signal.

### PCR 5' Nef from p17/24trNef1 using primers:

Polymerase = PWO (Roche) throughout.
Sense: S-Nef
   ATGGTGGGTTTTCCAGTCACACC [SEQ ID NO: 21]
   Antisense: ASNef-G:
   GATGAAATGCTAGGCGGCTGTCAAACCTC [SEQ ID NO: 22]
Cycle: 95°C(30s) then 15 cycles 95°C(30s), 55°C(30s), 72°C(60s), then 72°C(120s) and hold at 4°C

### PCR 3' Nef from p17/24trNef1 using primers:

Sense: SNEF-G
   GAGGTTTGACAGCCGCCTAGCATTTCATC [SEQ ID NO: 23]
Antisense: AStrNef
   CGCGGATCCTCAGCAGTTCTTGAAGTACTCC [SEQ ID NO: 24]

Cycle: 95°C(30s) then 15 cycles 95°C(30s), 55°C(30s), 72°C(60s), then 72°C(120s) and hold at 4°C

The PCR products were gel purified, and stitched using the 5' (S-Nef) and 3' (AstrNef) primers.
Cycle: 95°C(30s) then 15 cycles 95°C(30s), 55°C(30s), 72°C(60s), then 72°C(180s) and hold at 4°C.
The PCR product was PCR cleaned, cut with BglII / BamHI, and the 367bp fragment gel purified and cloned into BglII/BamHI cut gel purified p73i-GN.

### Example 12

### Plasmid: p73I-RT w229k (Inactivated RT)

### Gene of Interest:

Generation of an inactivated RT gene downstream of an Iowa length HCMV promoter + exon 1, and upstream of a rabbit β-globin poly-adenylation signal.

Due to concerns over the use of an active HIV RT species in a therapeutic vaccine inactivation of the gene was desirable. This was achieved by PCR mutagenesis of the RT (derived from P73I-GRN2) amino acid position 229 from Trp to Lys (R7271 p1-28).

### Primers:

PCR 5' RT + mutation using primers:
(polymerase = PWO (Roche) throughout)

### Sense : RT3-u:1

GAATTCGCGGCCGCGATGGGCCCCATCAGTCCCATCGAGACCGTGCCGGTGAAGCTG
AAACCCGGGAT [SEQ ID NO: 25]

Antisense: AScoRT-Trp229Lys
   GGAGCTCGTAGCCCATCTTCAGGAATGGCGGCTCCTTCT [SEQ ID NO: 26]
Cycle:
1 x [94°C (30s)]
15 x [94°C (30s) /55°C (30s) /72°C (60s)]
1 x [72°C (180s)]
PCR gel purify

### PCR 3' RT + mutation using primers:

Antiense: RT3- 1:1 
GAATTCGGATCCTTACAGCACCTTTCTAATCCCCGCACTCACCAGCTTGTCGACCTG
CTCGTTGCCGC [SEQ ID NO: 27]
Sense: ScoRT-Trp229Lys
   CCTGAAGATGGGCTACGAGCTCCATG [SEQ ID NO: 28]

### Cycle:

1 x [94°C (30s)]
15 x [94°C (30s) /55°C (30s) /72°C (60s)]
1 x [72°C (180s)]
PCR gel purify

The PCR products were gel purified and the 5' and 3' ends of RT were stitched using the 5' (RT3-U1) and 3' (RT3-L1) primers.

### Cycle:

1 x [94°C (30s) ]
15 x [94°C (30s) /55°C (30s) /72°C (120s)]
1 x [72°C (180s)]

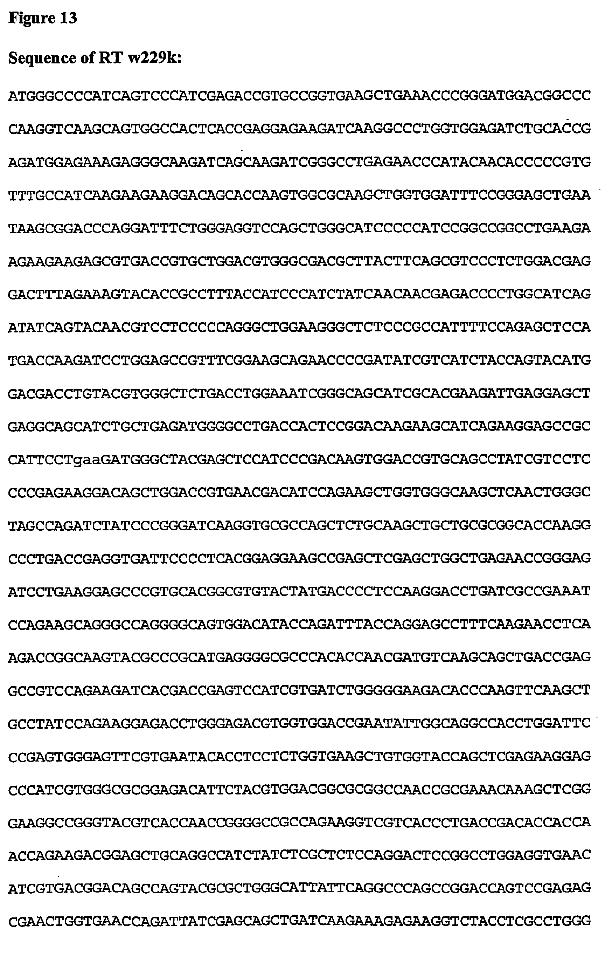

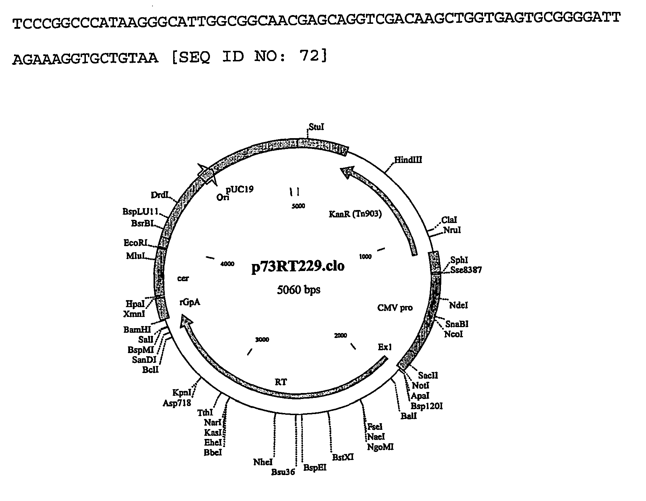

The PCR product was gel purified, and cloned into p7313ie, utilising NotI and BamHI restriction sites, to generate p73I-RT w229k. (See figure 13)

### Example 13:

### Plasmid: p73i-Tgrn (#3)

### Gene of interest:

The p17/p24 portion of the codon optimised gag, codon optimised RT and truncated Nef gene from the HIV-1 clade B strain HXB2 downstream of an Iowa length HCMV promoter + exon1, and upstream of a rabbit β-globin poly-adenylation signal.

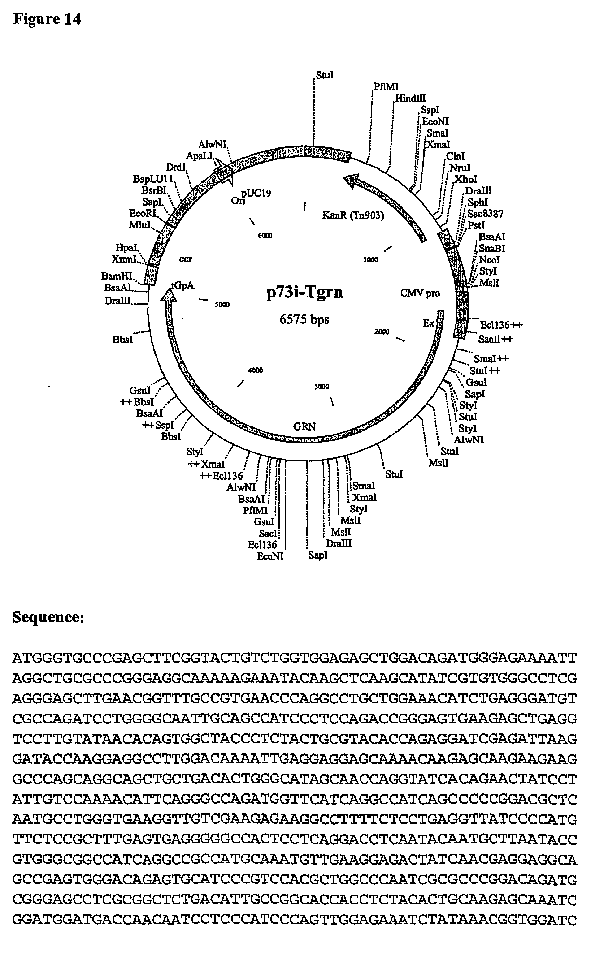

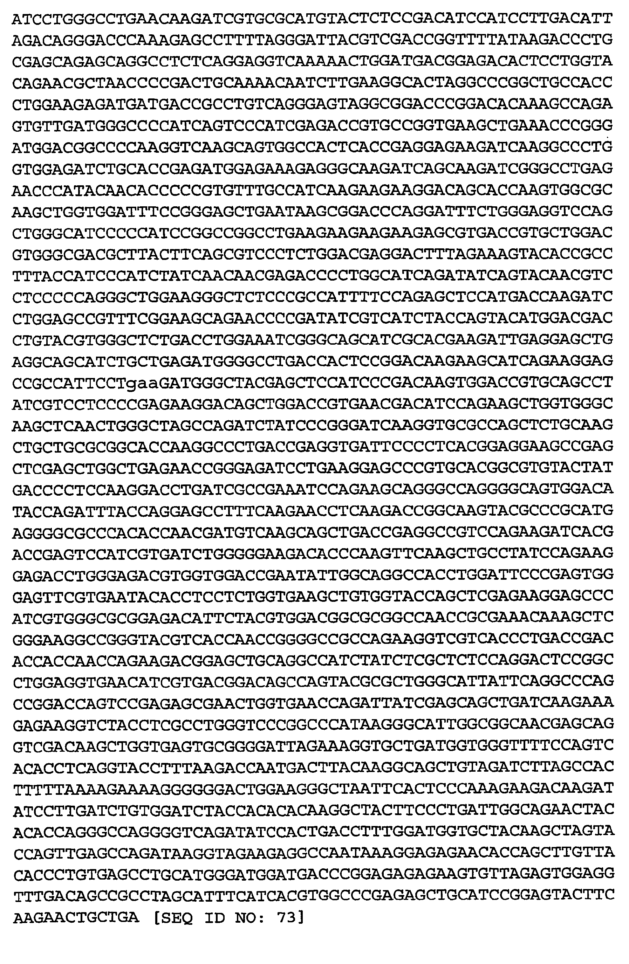

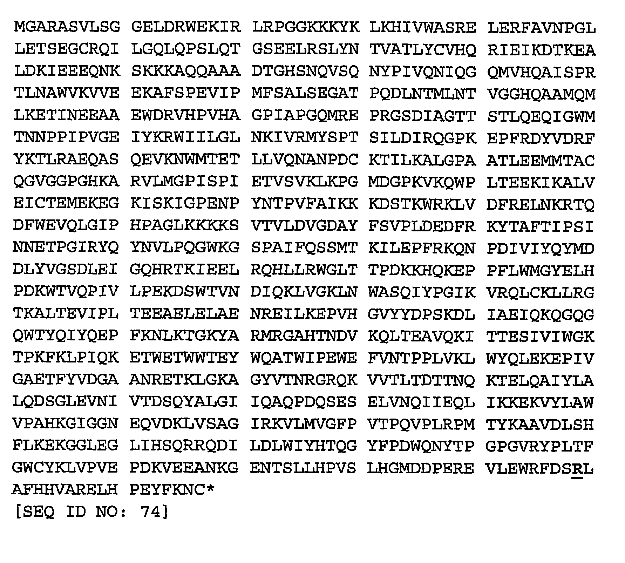

Triple fusion constructs which contain an active form of RT, may not be acceptable to regulatory authorities for human use thus inactivation of RT was achieved by Insertion of a NheI and ApaI cut fragment from p73i-RT w229k, into NheI/ApaI cut p73i-GRN2#19 (Figure 14). This results in a W → K change at position 229 in RT.

### Example 14

### p73I-Tnrg (#16)

### Gene of interest:

The truncated Nef , inactivated codon optimised RT and p17/p24 portion of the codon optimised gag gene from the HIV-1 clade B strain HXB2 downstream of an Iowa length HCMV promoter + exon1, and upstream of a rabbit β-globin poly-adenylation signal.

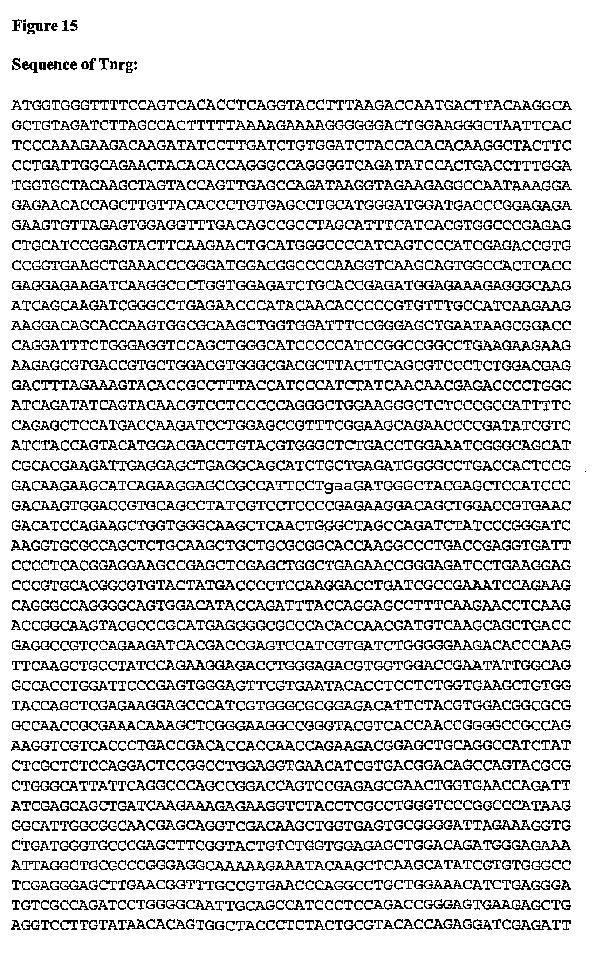

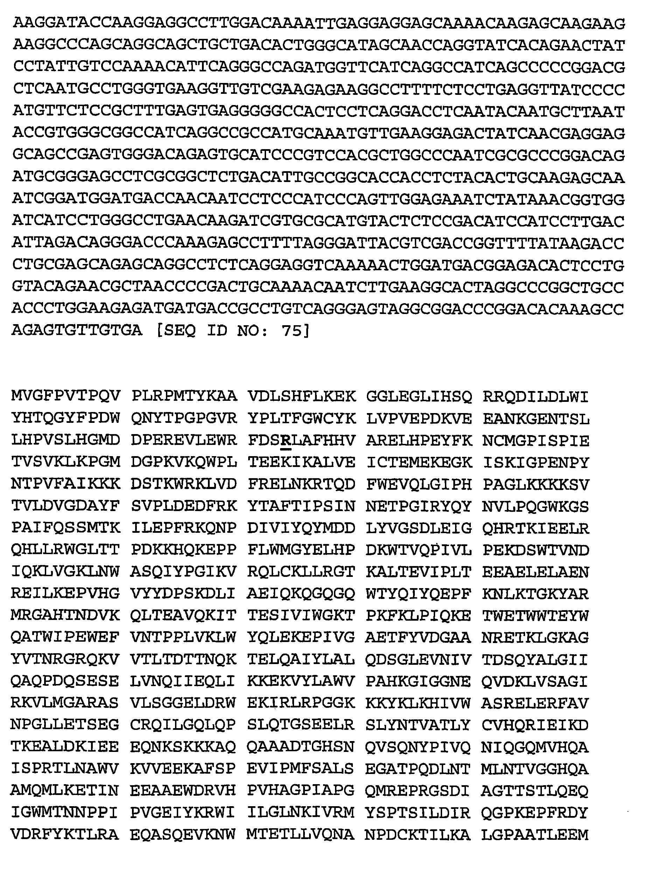

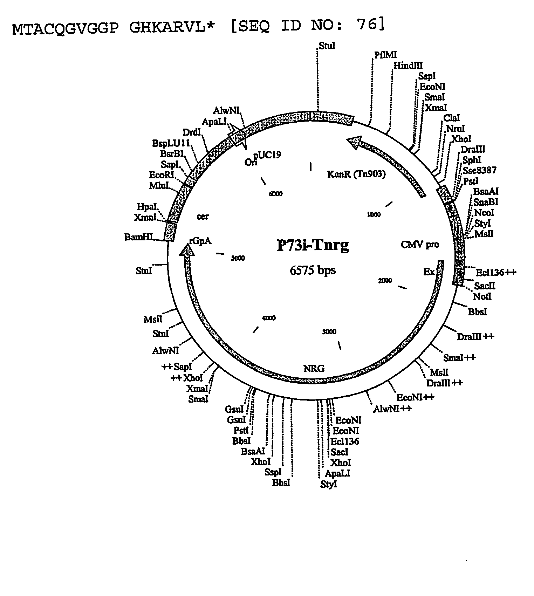

The order of the genes in the polyprotein encoded by p73i-Tgrn were rearranged by PCR and PCR stitching to generate p73I-Tnrg (Figure 15). Each gene was PCR amplified and gel purified prior to PCR stitching of the genes to form a single polyprotein. The product was gel purified, NotI/BamHI digested and ligated into NotI/BamHI cut p7313ie.

### Primers:

### trNef PCR

S-Nef (Not I)
   CATTAGAGCGGCCGCGATGGTGGGTTTTCCAC [SEQ ID NO: 29]
AS-Nef-coRT linker

GATGGGACTGATGGGGCCCATGCAGTTCTTGAACTACTCCGG [SEQ ID NO:
30]

### RTw229k PCR

S-coRT
   ATGGGCCCCATCAGTCCCATCGAG [SEQ ID NO: 31]
AS-coRT-p17p24 linker

CAGTACCGAAGCTCGGGCACCCATCAGCACCTTTCTAATCCCCGC [SEQ ID NO:
32]

### p17p24opt PCR

S-p17p24opt
   ATGGGTGCCCGAGCTTCGGTACTG [SEQ ID NO: 33]
AS-p17p24opt (BamHI)
   GATGGGGGATCCTCACAACACTCTGGCTTTGTGTCC [SEQ ID NO: 34]

PCR conditions for individual products and stitching using VENT DNA polymerase (NEB):
1 x [94°C (30s)]
25 x [94°C (30s) /55°C (30s) /72°C (120s [p17p24 or RT] or 60s [trNef])]
1 x [72°C (240s)]

The PCR products were gel purified and used in a PCR stitching utilising the primers S-trNef (NotI) and ASp17p24opt (*Bam*HI):
1 x [94°C (30s)
25 x [94°C (30s) /55°C (30s) /72°C (210s)]
1 x [72°C (240s)]

The 3000bp product was gel purified and cut with NotI and BamHI which was PCR cleaned and ligated into NotI/BamHI digested gel purified p7313ie to generate p73i-Tnrg.

### Example 15:

### 1. Plasmid: P73i-Tngr (#3)

### Gene of Interest:

The truncated Nef , p17/p24 portion of the codon optimised gag and inactivated codon optimised RT gene from the HIV-1 clade B strain HXB2 downstream of an Iowa length HCMV promoter + exon1, and upstream of a rabbit β-globin poly-adenylation signal.

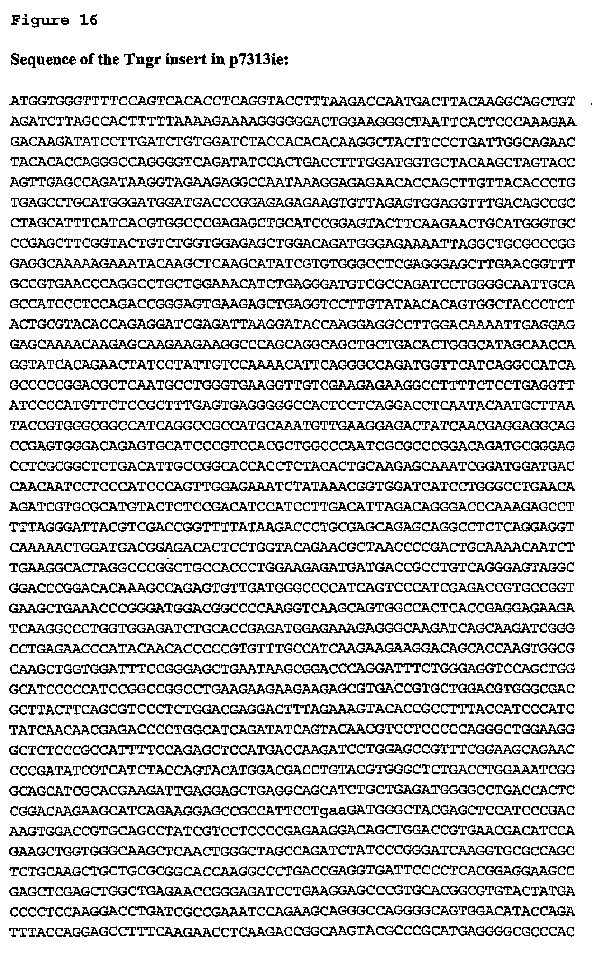

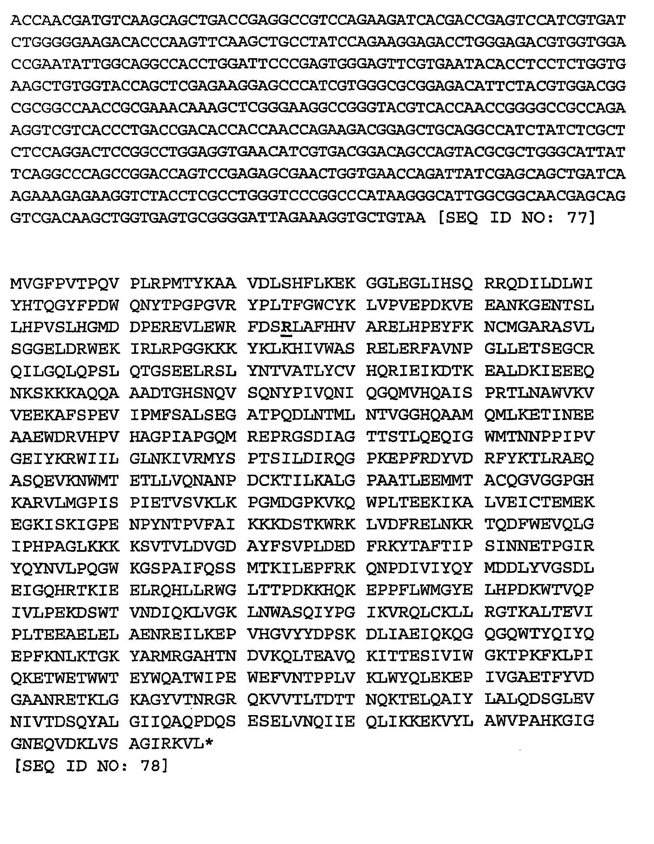

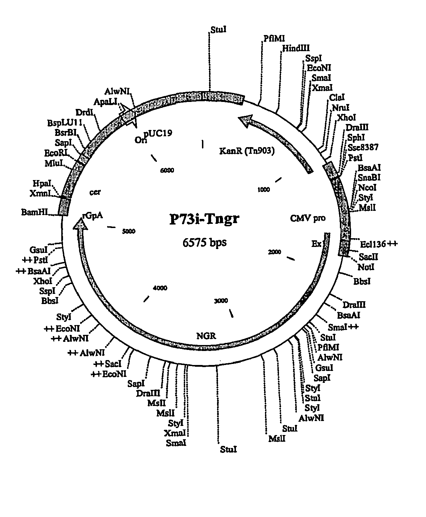

The order of the genes in the polyprotein encoded by p73i-Tgrn were rearranged by PCR to generate p73I-Tngr (Figure 16). Codon optimised p17/p24 and RT were generated as a single product, and PCR stitched to amplified trNef. The product was gel purified, NotI/BamHI digested and ligated into NotI/BamHI cut p7313ie.

### Primers:

### P17/p24 - RT 3'PCR:

Sp17p24opt (sense)
   ATGGGTGCCCGAGCTTCGGTACTG [SEQ ID NO: 35]
RT3 1:1 (antisense)

GAATTCGGATCCTTACAGCACCTTTCTAATCCCCGCACTCACCAGCTTGTCGACCTG
CTCGTTGCCGC [SEQ ID NO: 36]

### TrNef 5'PCR

S-Nef (NotI)
   CATTAGAGCGGCCGCGATGGTGGGTTTTCCAC [SEQ ID NO: 37]
AS-Nef-p17p24

CAGTACCGAAGCTCGGGCACCCATGCAGTTCTTGAACTACTCCGG [SEQ ID NO:
38]

PCR conditions for individual products and stitching using VENT DNA polymerase (NEB):
1 x [94°C (30s)]
25 x [94°C (30s) /55°C (30s) /72°C (180s [p17p24+RT] or 60s [trNef] or 210s [stitching])]
1 x [72°C (240s)]

The 3000bp product was gel purified and cut with NotI and BamHI which was PCR cleaned and ligated into NotI/BamHI digested gel purified p7313ie to generate p73i-Tngr.

### Example 16:

### Plasmid: p73I-Trgn (#6)

### Gene of interest:

The inactivated codon optimised RT, p17/p24 portion of the codon optimised gag and truncated Nef gene from the HIV-1 clade B strain HXB2 downstream of an Iowa length HCMV promoter + exon1, and upstream of a rabbit β-globin poly-adenylation signal.

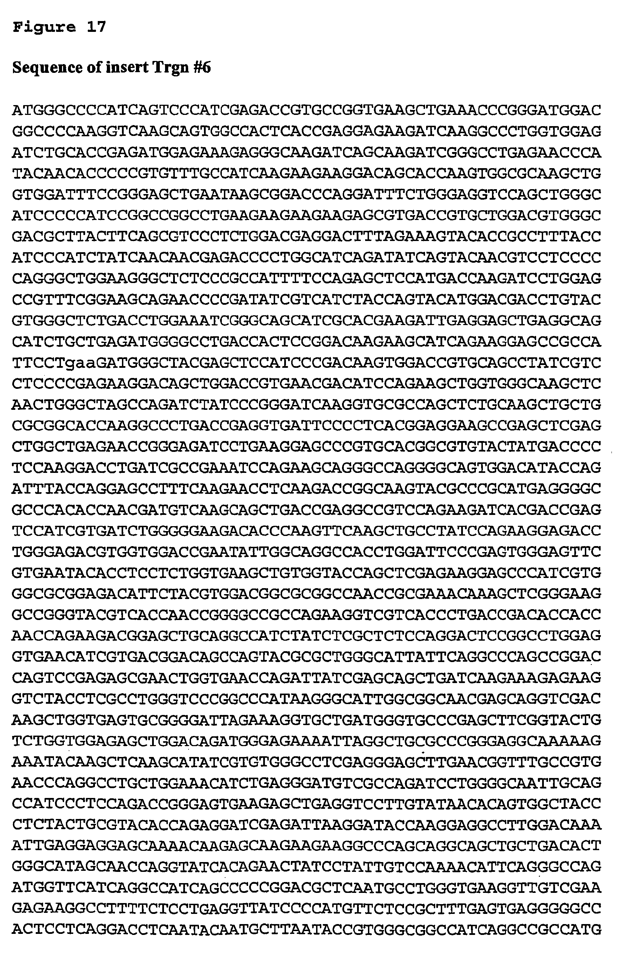

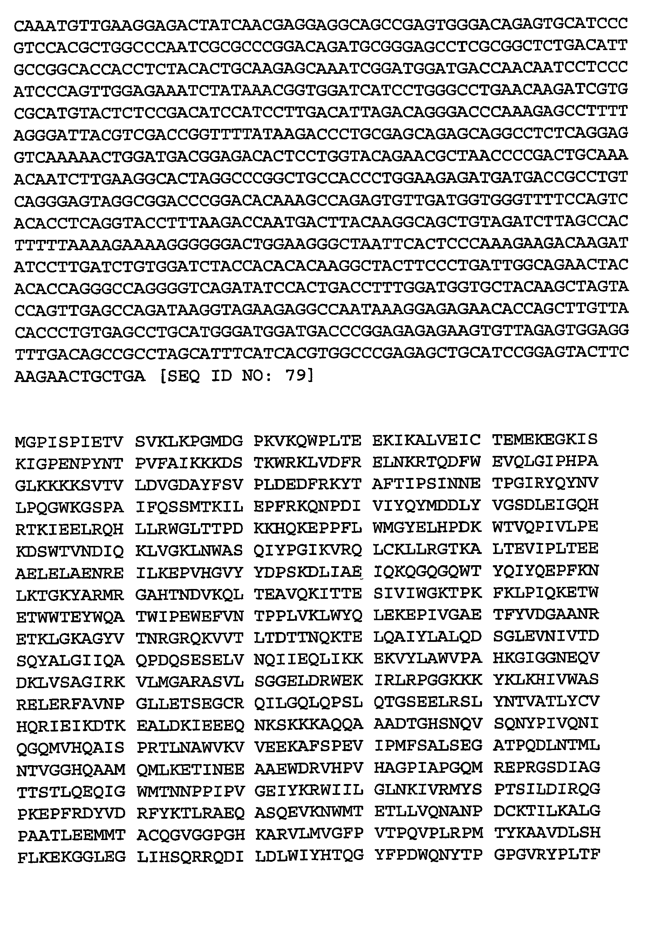

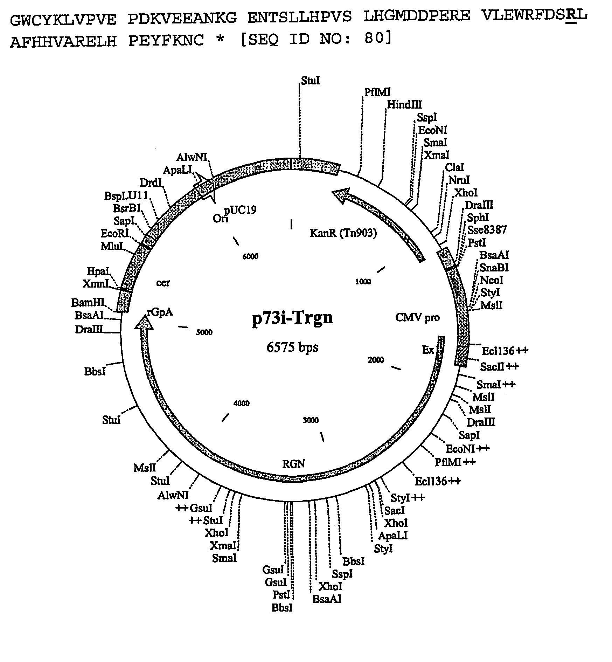

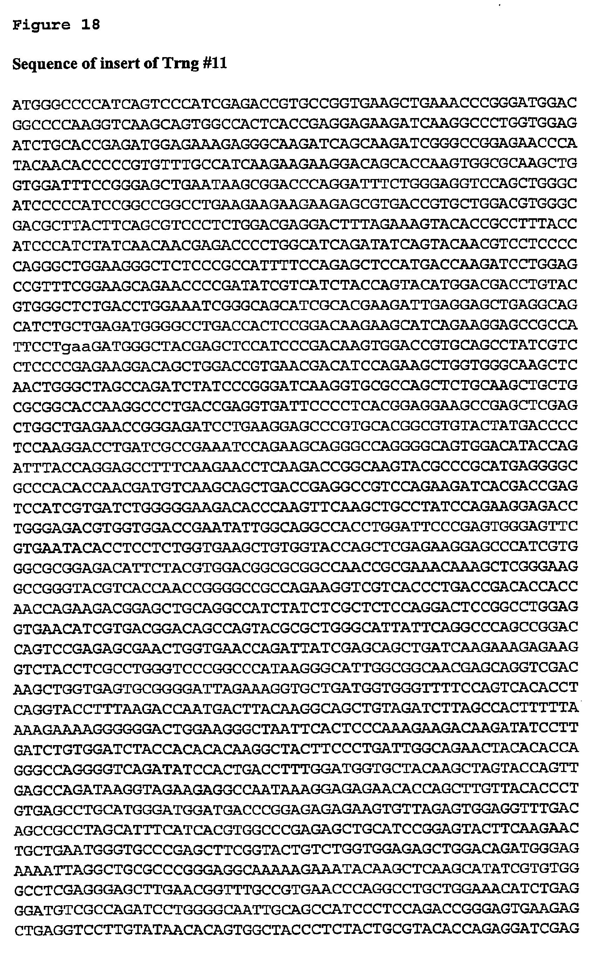

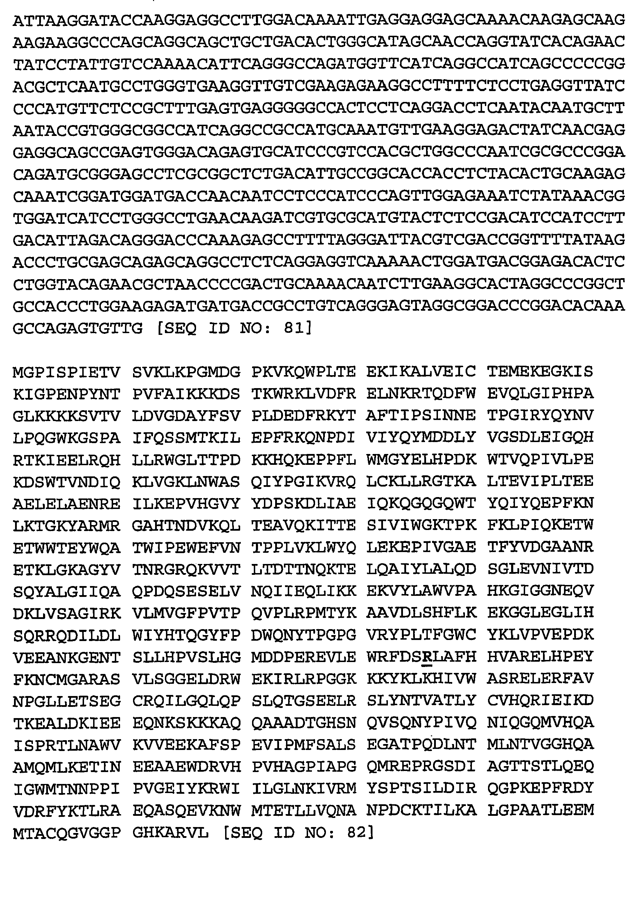

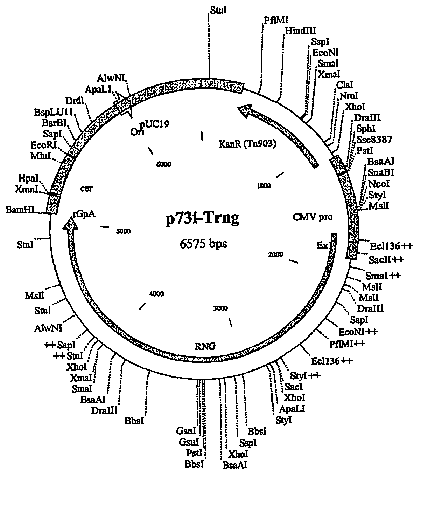

The order of the genes within the construct was achieved by PCR amplification of p17p24-trNef and RTw229k from the plasmids p73I-GN2 and p73I-RTw229k respectively. PCR stitching was performed and the product gel purified and NotI/BamHI cut prior to ligation with NotI/BamHI digested p7313ie. Sequencing revealed that p17p24 was not fully optimised a 700bp fragment was then AgeI/MunI cut from the coding region and replaced with MunI/Age fragment from p73i-Tgrn#3 containing the correct coding sequence. (See figure 17).

### Primers:

### p17p24-trNef PCR

S-p17p24opt
   ATGGGTGCCCGAGCTTCGGTACTG [SEQ ID NO: 39]

### AstrNef (BamHI)

### RTw229k

RT3-U:1 
GAATTCGCGGCCGCGATGGGCCCCATCAGTCCCATCGAGACCGTGCCGGTGAAGCTG
AAACCCGGGAT [SEQ ID NO: 40]
AS-coRT-p17p24opt linker

CAGTACCGAAGCTCGGGCACCCATCAGCACCTTTCTAATCCCCGC [SEQ ID NO:
41]

PCR conditions for individual products and stitching using VENT DNA polymerase (NEB):
1 x [94°C (30s)]
25 x [94°C (30s) /55°C (30s) /72°C (120s (PCR) or 180s (stitching)
1 x [72°C (240s)]

The 3000bp product from the PCR stitch was gel purified and cut with NotI and BamHI which was PCR cleaned and ligated into NotI/BamHI digested gel purified p7313ie to generate p73i-Tngr. Sequence analysis showed that p17p24 sequence obtained from p73I-GN2 was not fully codon optimised and that this had been carried over into the new plasmid. This was rectified by cutting a 700bp fragment from p73i-Tngr cut with MunI and AgeI, and replacing it by ligation with a 700bp MunI/AgeI digested product from p73i-Tgrn to generate the construct p73I-Tngr#6.

### Example 17:

### Plasmid: p73i-Trng (#11)

### Gene of Interest:

The inactivated codon optimised RT, truncated Nef and p17/p24 portion of the codon optimised gag gene from the HIV-1 clade B strain HXB2 downstream of an Iowa length HCMV promoter + exon1, and upstream of a rabbit β-globin poly-adenylation signal.

The order of the genes within the construct was achieved by PCR amplification of the RT-trNef and p17p24 genes from p73i-Tgrn. PCR stitching of the two DNA fragments was performed and the 3kb product gel purified and NotI/BamHI cut prior to ligation with NotI/BamHI digested p7313ie, and yielded p73I Trng (#11).

### Primers:

### RTw229k-trNef

RT3-u:1 
GAATTCGCGGCCGCGATGGGCCCCATCAGTCCCATCGAGACCGTGCCGGTGAAGCTG
AAACCCGGGAT [SEQ ID NO: 42]
AS-Nef-p17p24opt linker

CAGTACCGAAGCTCGGGCACCCATGCAGTTCTTGAACTACTCCGG [SEQ ID NO:
43]

### P17p24

S-p17p24opt
   ATGGGTGCCCGAGCTTCGGTACTG [SEQ ID NO: 44]
AS-p17p24opt(BamHI)
   GATGGGGGATCCTCACAACACTCTGGCTTTGTGTCC [SEQ ID NO: 45]

PCR conditions for individual products and stitching using VENT DNA polymerase (NEB):
1 x [94°C (30s)]
25 x [94°C (30s)/55°C (30s)/72°C (120s (PCR of genes) or 180s (stitching)
1 x [72°C (240s)]

The 3000bp product from the PCR stitch was gel purified and cut with NotI and BamHI which was PCR cleaned and ligated into NotI/BamHI digested gel purified p7313ie to generate p73i-Tngr.

### Example 18:

### p73i-Tgnr (#f1)

### Gene of interest:

The p17/p24 portion of the codon optimised gag, truncated Nef and codon optimised inactivated RT gene from the HIV-1 clade B strain HXB2 downstream of an Iowa length HCMV promoter + exon1, and upstream of a rabbit β-globin poly-adenylation signal.

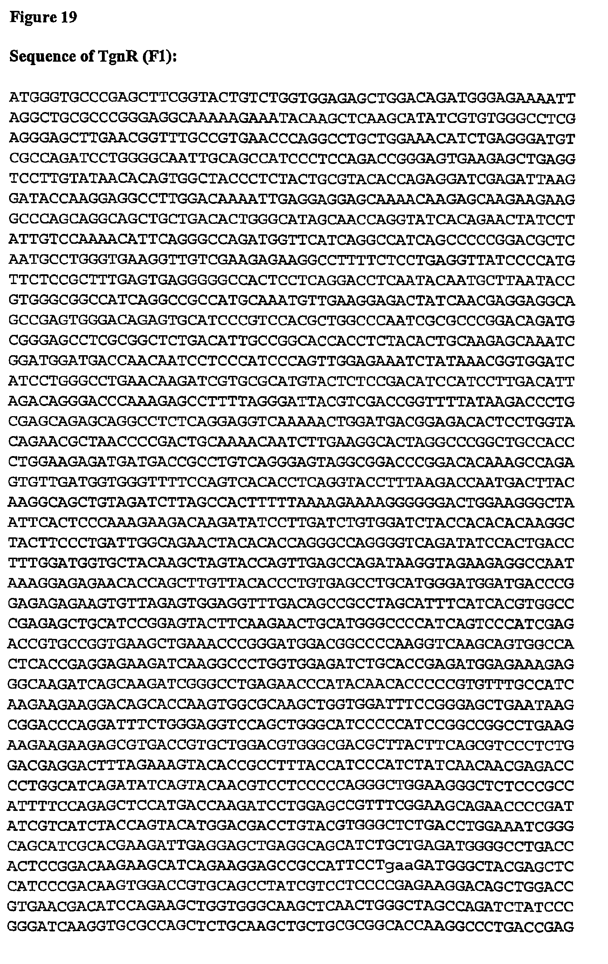

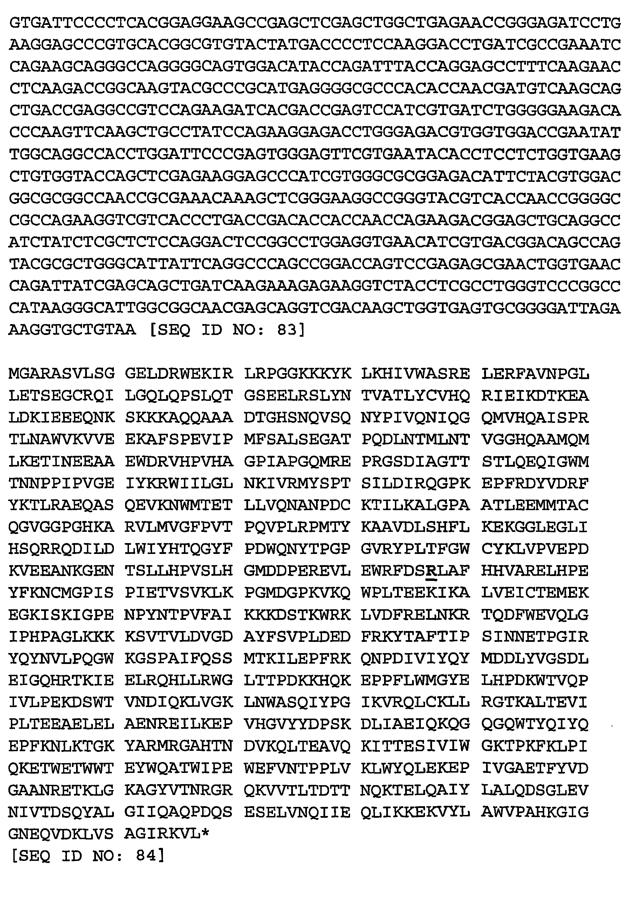

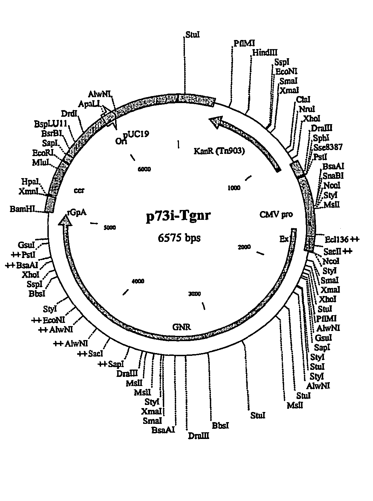

The order of the genes within the construct was achieved by PCR amplification of p17p24-trNef and RTw229k from the plasmids p73I-GN2 and p73I-RTw229k respectively. PCR stitching was performed and the product gel purified and NotI/BamHI cut prior to ligation with NotI/BamHI digested p7313ie. Two sequence errors were spotted in the sequence (p17p24 and RT) which were subsequently repaired by replacement with correct portions of the genes utilising restriction sites within the polyprotein. (See figure 19).

### Primers:

### p17p24-trNef PCR

S-p17p24opt
   ATGGGTGCCCGAGCTTCGGTACTG [SEQ ID NO: 46]
AS-Nef-coRTlinker

GATGGGACTGATGGGGCCCATGCAGTTCTTGAACTACTCCGG [SEQ ID NO:
47]

### RTw229k

S-coRT
   ATGGGCCCCATCAGTCCCATCGAG [SEQ ID NO: 48]
RT3-1:1

GAATTCGGATCCTTACAGCACCTTTCTAATCCCCGCACTCACCAGCTTGTCGACCTG
CTCGTTGCCGC [SEQ ID NO: 49]

PCR conditions for individual products and stitching using VENT DNA polymerase (NEB):
1 x [94°C (30s)]
25 x [94°C (30s)/55°C (30s)/72°C (120s (PCR) or 180s (stitching)
1 x [72°C (240s)]

The 3000bp product was gel purified and cut with NotI and BamHI which was PCR cleaned and ligated into NotI/BamHI digested gel purified p7313ie to generate p73i-Tngr. Sequencing revealed that p17p24 was not fully optimised a 700bp fragment was subsequently AgeI/MunI cut from the coding region and replaced with MunI/Age fragment from p73i-Tgrn#3 containing the correct coding sequence. The polyprotein also contained a single point mutation (G2609A) resulting in an amino acid substitution of Thr to Ala in the RT portion of the polyprotein. The mutation was corrected by ApaI/BamHI digestion of the construct and PCR clean up to remove the mutated sequence, which was replaced by ligation with an ApaI/BamHI digested portion of RT from p73i-Tgnr.

### Example 19:

### Preparation of plasmid-coated 'gold slurry' for 'gene gun' DNA cartridges

Plasmid DNA (approximately 1µg/µl), eg. 100 ug, and 2µm gold particles, eg. 50 mg, (PowderJect), were suspended in 0.05M spermidine, eg. 100 ul, (Sigma). The DNA was precipitated on to the gold particles by addition of 1M CaCl₂, eg. 100ul (American Pharmaceutical Partners, Inc., USA). The DNA/gold complex was incubated for 10 minutes at room temperature, washed 3 times in absolute ethanol, eg. 3 x 1 ml, (previously dried on molecular sieve 3A (BDH)). Samples were resuspended in absolute ethanol containing 0.05mg/ml of polyvinylpyrrolidone (PVP, Sigma), and split into three equal aliquots in 1.5 ml microfuge tubes, (Eppendorf). The aliquots were for analysis of (a) 'gold slurry', (b) eluate- plasmid eluted from (a) and (c) for preparation of gold/ plasmid coated Tefzel cartridges for the 'gene gun', (see Example 3 below). For preparation of samples (a) and (b), the tubes containing plasmid DNA / 'gold slurry' in ethanol / PVP were spun for 2 minutes at top speed in an Eppendorf 5418 microfuge, the supernatant was removed and the 'gold slurry' dried for 10 minutes at room temperature. Sample (a) was resuspended to 0.5 - 1.0 ug / ul of plasmid DNA in TE pH 8.0, assuming approx. 50 % coating. For elution, sample (b) was resuspended to 0.5 - 1.0 ug / ul of plasmid DNA in TE pH 8.0 and incubated at 37°C for 30 minutes, shaking vigorously, and then spun for 2 minutes at top speed in an Eppendorf 5418 microfuge and the supernatant, eluate, was removed and stored at -20°C. The exact DNA concentration eluted was determined by spectrophotometric quantitation using a Genequant II (Pharmacia Biotech).

### Example 20:

### Preparations of Cartridges for DNA immunisation

Preparation of cartridges for the Accell gene transfer device was as previously described (Eisenbraun et al DNA and Cell Biology, 1993 Vol 12 No 9 pp 791-797; Pertner et al). Briefly, plasmid DNA was coated onto 2 µm gold particles (DeGussa Corp., South Plainfield, N.J., USA) and loaded into Tefzel tubing, which was subsequently cut into 1.27 cm lengths to serve as cartridges and stored desiccated at 4°C until use. In a typical vaccination, each cartridge contained 0.5 mg gold coated with a total of 0.5 µg DNA/cartridge.

### Example 21:

### Immune Response to HIV antigens following DNA vaccination utilising the gene gun.

Mice (n=3/group) were vaccinated with antigens encoded by nucleic acid and located in two vectors. P7077 utilises the HCMV IE promoter including Intron A and exon 1 (fcmv promoter). P73I delivers the same antigen, but contains the HCMV IE promoter (icmv promoter) that is devoid of Intron A, but includes exon 1.

Plasmid was delivered to the shaved target site of abdominal skin of F1 (C3H x Balb/c) mice. Mice were given a primary immunisation of 2 x 0.5 µg DNA on day 0, boosted with 2 x 0.5 µg DNA on day 35 and cellular response were detected on day 40 using IFN - gamma Elispot.
- P73I - empty vector
- P7077 - empty vector
- P7077 GRN - (f CMV promoter) Gag, RT, Nef
- P73I GRN - (i CMV promoter) Gag, RT, Nef
- P73I GR3N - (CMV promoter) Optimised Gag, Optimised RT, Nef
- P7077 GN - (f CMV promoter) Gag, Nef
- P73I GN - (i CMV promoter) Gag, Nef Cytotoxic T Cell Responses

The cytotoxic T cell response was assessed by CD8+ T cell-restricted IFN-γ ELISPOT assay of splenocytes collected 5 days later. Mice were killed by cervical dislocation and spleens were collected into ice-cold PBS. Splenocytes were teased out into phosphate buffered saline (PBS) followed by lysis of red blood cells (1 minute in buffer consisting of 155mM NH₄Cl, 10 mM KHCO₃, 0.1mM EDTA). After two washes in PBS to remove particulate matter the single cell suspension was aliquoted into ELISPOT plates previously coated with capture IFN-γ antibody and stimulated with CD8-restricted cognate peptide (Gag, Nef or RT). After overnight culture, IFN-γ producing cells were visualised by application of anti-murine IFN-γ-biotin labelled antibody (Pharmingen) followed by streptavidin -conjugated alkaline phosphatase and quantitated using image analysis.

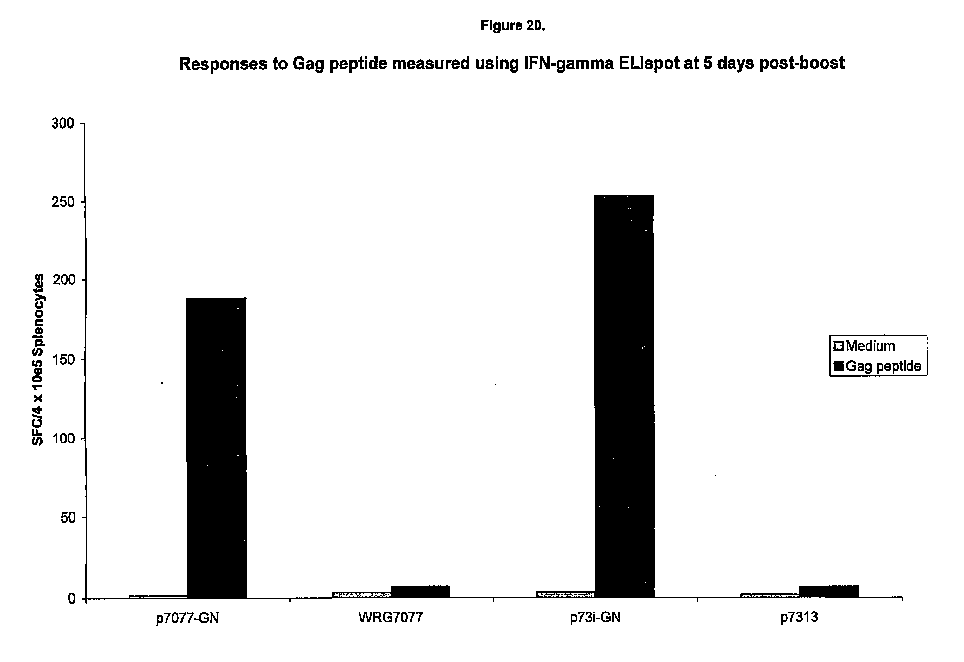

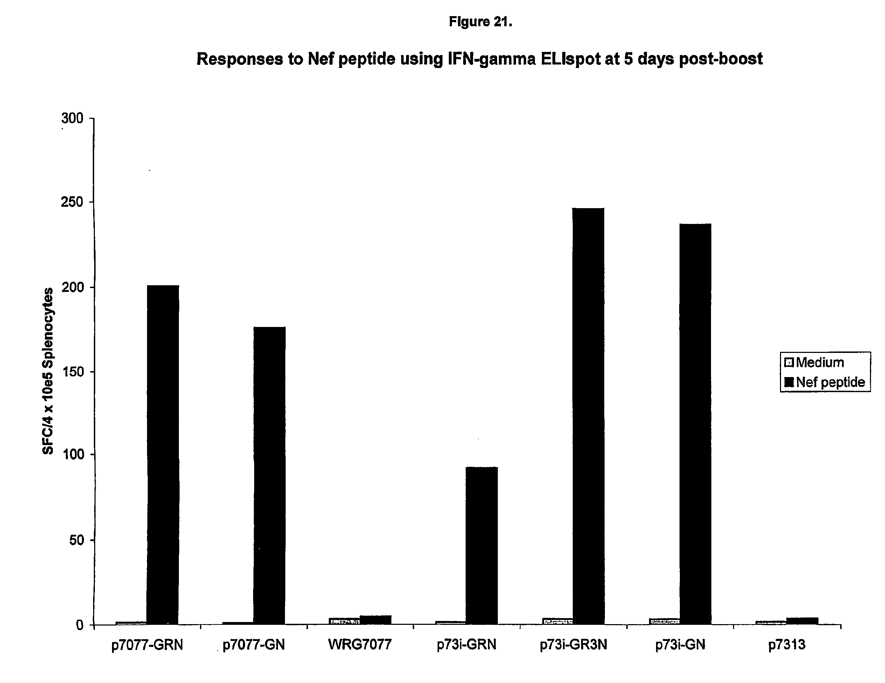

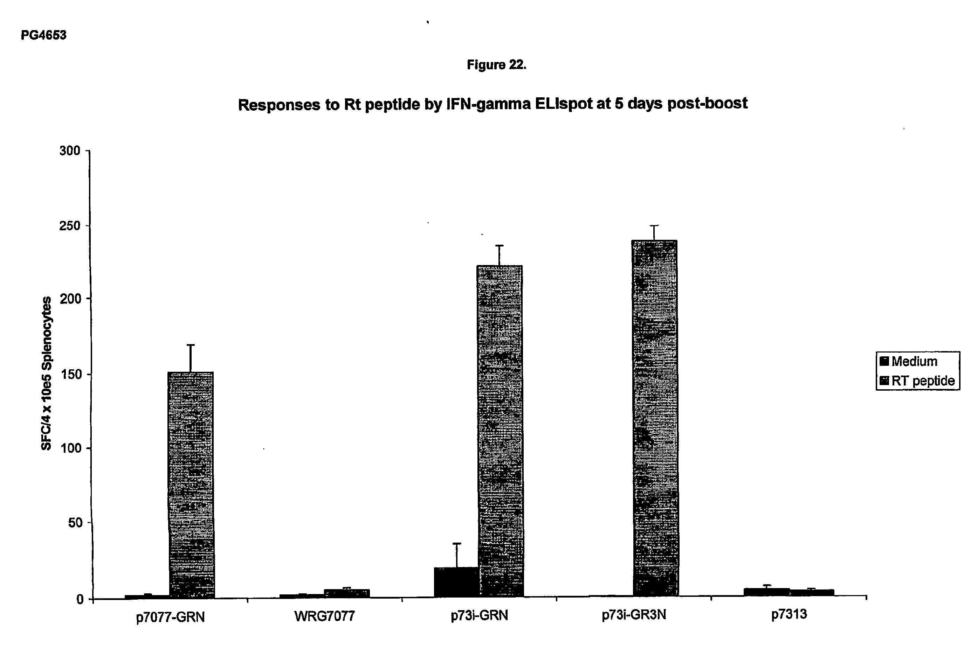

The result of this experiment are shown in figures 20, 21, and 22.

### Example 22:

### Immunogenicity of Vaccine Constructs

### 1. Cellular Assays

The cellular immune response comprises cytotoxic CD8 cells and helper CD4 cells. A sensitive method to detect specific CD8 and CD4 cells is the ELIspot assay which can be used to quantify the number of cells capable of secreting interferon-y or IL-2. The ELIspot assay relies on the capture of cytokines secreted from individual cells. Briefly, specialised microtitre plates are coated with anti-cytokine antibodies. Splenocytes isolated from immunised animals are incubated overnight in the presence of specific peptides representing known epitopes (CD8) or proteins (CD4). If cells are stimulated to release cytokines they will bind to the antibodies on the surface of the plate surrounding the locality of the individual producing cells. Cytokines remain attached to the coating antibody after the cells have been lysed and plates washed. The assay is developed in a similar way to an ELISA assay using a biotin/avidin amplification system. The number of spots equates to the number of cytokine producing cells.

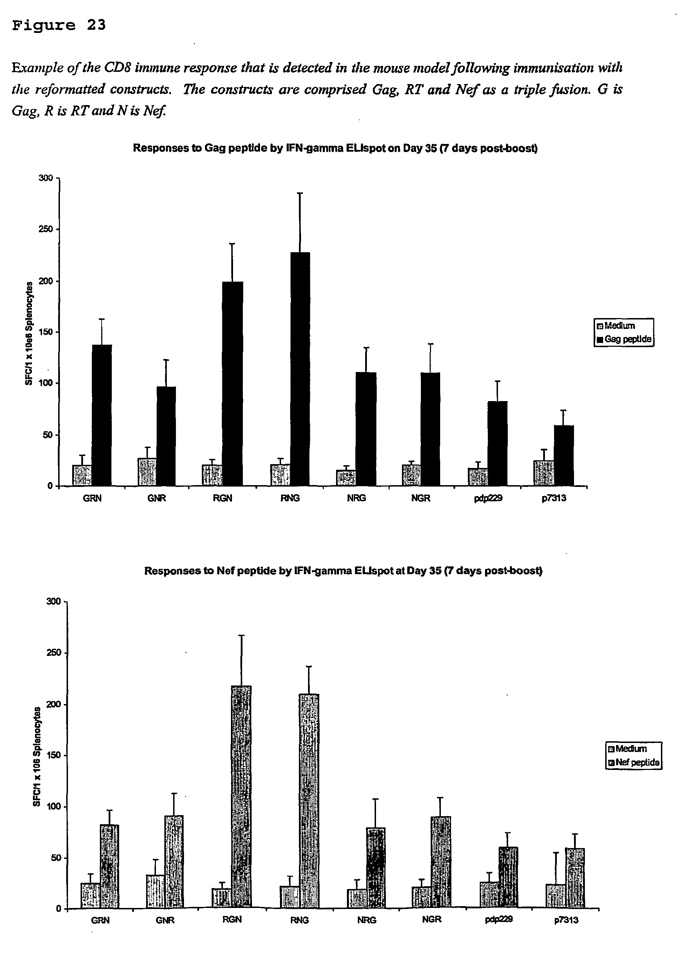

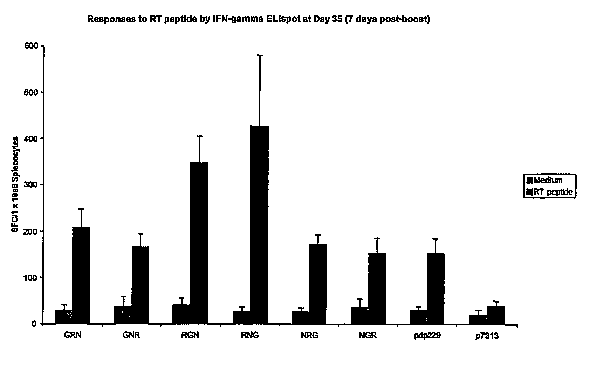

CD8 responses to the following K2^{d}-restricted murine epitopes: Gag (AMQMLKETI), Nef (MTYKAAVDL) and RT (YYDPSKDLI) and CD4 responses to Gag and RT proteins were recorded for all 6 constructs. The results of these assays were analysed statistically and constructs were ranked according to their immunogenicity. The result is shown in figure 23 of the figures.

### 2. Humoral Assays

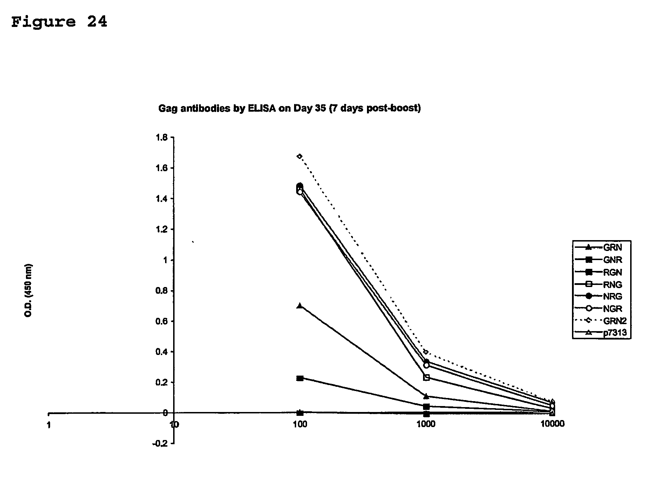

Blood samples were collected for antibody analysis at 7 and 14 days post-boost from two experiments. Serum was separated and stored frozen until antibody titres could be measured using specific ELISA assays. All samples were tested for antibodies to Gag, Nef and RT. Briefly, ELISA plates were coated with the relevant protein. Excess protein was washed off before diluted serum samples were incubated in the wells. The serum samples were washed off and anti-mouse antiserum conjugated to an appropriate tag was added. The plate was developed and read on a plate reader. The results are shown in figure 24.

### 3. Antibody Data

Antibody titres were measured for all six constructs in four experiments. Construct p73i-GNR consistently generated no antibody responses to Gag and limited antibody responses to Nef. The reason for this is unclear, as T-cell responses were observed from splenocytes isolated from the same mice, indicating that the Gag protein was being expressed in vivo.

The ranking for the generation of Gag specific antibodies was:

### RNG>GRN>NRG>RGN>NGR>GNR

### Analysis cellular immunology data

The objective was to rank the 6 constructs on the basis of spot count data from 3 immunology experiments. Three sets of responses were assessed:
CD8 responses to Gag, Nef and RT at Day 7 (7 days post primary),
CD4 responses to Gag and RT at Day 35 (7 days post boost),
CD8 responses to Gag, Nef and RT at Day 35 (7 days post boost).

Each response (e.g. CD8 response to Gag) was modeled using a linear mixed effect model in SAS version 8. The model included fixed effects of construct, whether the particular antigen (Gag, Nef or RT) was present or absent, and whether IL-2 was present or absent. In addition, for CD8 responses, where data were available from each individual mouse, subject was included as a random effect in the model. The model included interaction terms to allow for a different effect of construct for each combination of the antigen (present/absent) and IL-2 (present/absent).

From the model, the difference in adjusted mean response between each construct and p7313 (the control group) was estimated separately for each combination of antigen (present/absent) and IL-2 (present/absent), together with a p-value indicating whether the difference was statistically significant. Based on the differences and p-values in the presence of the antigen and the absence of IL-2, constructs were ranked, by assigning a score of 6 to the construct with the largest difference, 5 to the next largest, etc, but 0 to any constructs where the difference was not statistically significant at the 5% level.

The assumptions of the model - that the residuals were normally distributed with constant variance, were assessed using graphical methods and sensitivity analyses, where first a log and second a square root transformation of the response was modeled. The ranking of the constructs was not sensitive to departures from the assumptions of the model.

Having calculated the ranks for each response in each experiment separately, total ranks for the 3 sets of responses were calculated across all 3 experiments. The following table shows the total rankings across the 3 experiments.

**Total rankings of constructs for each of 3 sets of responses, combined across 3 immunology experiments.**

| **Construct** | **Day 7 (7 days post primary)** | **Day 35 (7 days post boost)** | |
|---|---|---|---|
| | CD8 | CD4 | CD8 |
| GRN | 5 | 18 | 3 |
| GNR | 17 | 24 | 28 |
| RGN | 28 | 23 | 33 |
| RNG | 25 | 27 | 37 |
| NRG | 25 | 19 | 0 |
| NGR | 4 | 14 | 10 |

RNG has the highest ranking for both sets of responses at Day 35, and the second highest ranking behind RGN at Day 7. RGN also receives high rankings for both sets of responses at Day 35.

## Claims

1. A nucleotide sequence operably linked to a heterologous promoter, wherein the nucleotide sequence encodes codon optimised RT, Nef truncate, codon optimised p17 Gag and codon optimised p24 Gag in the order RT, Nef, p17 Gag, p24 Gag.

2. A nucleotide sequence as claimed in claim 1 wherein the gag sequence is codon optimised to resemble the codon usage in a highly expressed human gene and the nucleotide sequence has an RSCU value of greater than 0.5.

3. A nucleotide sequence as claimed in either claim 1 or 2 wherein the RT is codon optimised to resemble a highly expressed human gene.

4. A nucleotide sequence as claimed in any preceding claim wherein the heterologous promoter is the promoter from HCMV IE gene.

5. A nucleotide sequence as claimed in claim 4 wherein the 5' of the promoter comprises exon 1.

6. A nucleotide sequence as claimed in any preceding claim wherein the RT encodes a mutation to substantially inactivate any reverse transcriptase activity.

7. A nucleotide sequence as claimed in claim 6 which the RT is mutated by substituting tryptophan 229 for Lysine.

8. A vector comprising a nucleotide sequence as claimed in any one of claims 1 to 7.

9. A vector as claimed in claim 8, which is a viral vector.

10. A viral vector as claimed in claim 9 which is a replication defective adenovirus.

11. A vector as claimed in claim 9 which is a double stranded DNA plasmid.

12. A protein encoded by a nucleotide sequence as claimed in any of claims 1 to 7.

13. A pharmaceutical composition comprising a nucleotide sequence of claim 1 to 7 or a vector of claim 8-11 and a pharmaceutically acceptable excipient, diluent, carrier or adjuvant.

14. A pharmaceutical composition as claimed in claim 13 adapted for intra-muscular or intra-dermal delivery.

15. A pharmaceutical composition as claimed in claim 13 or 14 wherein the carrier is a gold bead.

16. An intra-dermal delivery device comprising a pharmaceutical composition according to any one of claims 13-15.

17. A nucleotide sequence of any of claims 1 to 7, a vector of claim 8 to 11, or a composition of any of claims 13 to 15, for use in medicine.

18. A process for the production of a nucleotide as claimed in any of claims 1 to 7 comprising operably linking a nucleotide sequence encoding RT (codon optimised), Nef truncate, p17 Gag (codon optimised) and p24 Gag (codon optimised) in the order RT, Nef, p17 Gag, p24 Gag to a heterologous promoter.

## Patentansprüche

1. Nucleotidsequenz, die funktionell mit einem heterologen Promotor verknüpft ist, wobei die Nucleotidsequenz für eine Kodon-optimierte RT, eine Nef-Trunkierung, ein Kodon-optimiertes p17-Gag und ein Kodon-optimiertes p24-Gag in der Reihenfolge RT, Nef, p17-Gag, p24-Gag kodiert.

2. Nucleotidsequenz gemäß Anspruch 1, wobei die gag-Sequenz Kodon-optimiert ist, um der Kodonverwendung in einem hoch exprimierten Humangen ähnlich zu sein, und die Nucleotidsequenz einen RSCU-Wert von größer als 0,5 aufweist.

3. Nucleotidsequenz gemäß Anspruch 1 oder 2, wobei die RT Kodon-optimiert ist, um einem hoch exprimierten Humangen ähnlich zu sein.

4. Nucleotidsequenz gemäß einem der vorangehenden Ansprüche, wobei der heterologe Promotor der Promotor des HCMV-IE-Gens ist.

5. Nucleotidsequenz gemäß Anspruch 4, wobei das 5'-Ende des Promotors Exon 1 umfasst.

6. Nucleotidsequenz gemäß einem der vorangehenden Ansprüche, wobei die RT für eine Mutation kodiert, um im wesentlichen jegliche Reverse-Transkriptase-Aktivität zu inaktivieren.

7. Nucleotidsequenz gemäß Anspruch 6, wobei die RT durch Ersetzen von Lysin durch Tryptophan 229 mutiert ist.

8. Vektor, der eine Nucleotidsequenz gemäß einem der Ansprüche 1 bis 7 umfasst.

9. Vektor gemäß Anspruch 8, der ein viraler Vektor ist.

10. Viraler Vektor gemäß Anspruch 9, der ein Replikationsdefekter Adenovirus ist.

11. Vektor gemäß Anspruch 9, der ein doppelstängiges DNA-Plasmid ist.

12. Protein, das durch eine Nucleotidsequenz gemäß einem der Ansprüche 1 bis 7 kodiert wird.

13. Pharmazeutische Zusammensetzung, die eine Nucleotidsequenz gemäß Anspruch 1 bis 7 oder einen Vektor gemäß Anspruch 8 bis 11 und einen/ein pharmazeutisch annehmbare(n(s)/annehmbares Exzipienten, Verdünnungsstoff, Träger oder Adjuvans umfasst.

14. Pharmazeutische Zusammensetzung gemäß Anspruch 13, die für die intra-muskuläre oder intra-dermale Verabreichung angepasst ist.

15. Pharmazeutische Zusammensetzung gemäß Anspruch 13 oder 14, wobei der Träger eine Goldperle ist.

16. Intra-dermale Verabreichungsvorrichtung, umfassend eine pharmazeutische Zusammensetzung gemäß einem der Ansprüche 13 bis 15.

17. Nucleotidsequenz gemäß einem der Ansprüche 1 bis 7, Vektor gemäß Anspruch 8 bis 11 oder Zusammensetzung gemäß einem der Ansprüche 13 bis 15 zur Verwendung in der Medizin.

18. Verfahren zur Herstellung eines Nucleotids gemäß einem der Ansprüche 1 bis 7, umfassend die funktionelle Verknüpfung einer Nucleotidsequenz, die für eine RT (Kodon-optimiert), Nef-Trunkierung, p17-Gag (Kodon-optimiert) und p24-Gag (Kodon-optimiert) in der Reihenfolge RT, Nef, p17-Gag, p24-Gag kodiert, mit einem heterologen Promotor.

## Revendications

1. Séquence de nucléotides liée de manière opérationnelle à un promoteur hétérologue, dans laquelle la séquence de nucléotides code pour une RT à codons optimisés, un produit de troncature Nef, p17 Gag à codons optimisés et p24 Gag à codons optimisés dans l'ordre RT, Nef, p17 Gag, p24 Gag.

2. Séquence de nucléotides selon la revendication 1, dans laquelle la séquence Gag est à codons optimisés pour ressembler à l'usage de codon dans un gène humain hautement exprimé et la séquence de nucléotides a une valeur RSCU supérieure à 0,5.

3. Séquence de nucléotides selon l'une quelconque des revendications 1 ou 2, dans laquelle la RT est à codons optimisés pour ressembler à un gène humain hautement exprimé.

4. Séquence de nucléotides selon l'une quelconque des revendications précédentes, dans laquelle le promoteur hétérologue est le promoteur provenant du gène IE du CMVH.

5. Séquence de nucléotides selon la revendication 4, dans laquelle le 5' du promoteur comprend l'exon 1.

6. Séquence de nucléotides selon l'une quelconque des revendications précédentes, dans laquelle la RT code pour une mutation afin d'inactiver sensiblement toute activité de transcriptase inverse.

7. Séquence de nucléotides selon la revendication 6, dans laquelle la RT est mutée en remplaçant de la lysine par du tryptophane 229.

8. Vecteur comprenant une séquence de nucléotides telle que revendiquée dans l'une quelconque des revendications 1 à 7.

9. Vecteur selon la revendication 8, qui est un vecteur viral.

10. Vecteur viral selon la revendication 9, qui est un adénovirus à réplication défective.

11. Vecteur selon la revendication 9, qui est un plasmide d'ADN double brin.

12. Protéine codée par une séquence de nucléotides telle que revendiquée dans l'une quelconque des revendications 1 à 7.

13. Composition pharmaceutique comprenant une séquence de nucléotides des revendications 1 à 7 ou un vecteur des revendications 8 à 11, et un excipient, diluant, support ou adjuvant pharmaceutiquement acceptable.

14. Composition pharmaceutique selon la revendication 13, adaptée pour une administration intramusculaire ou intradermique.

15. Composition pharmaceutique selon la revendication 13 ou 14, dans laquelle le support est une bille d'or.

16. Dispositif d'administration intradermique comprenant une composition pharmaceutique selon l'une quelconque des revendications 13 à 15.

17. Séquence de nucléotides selon l'une quelconque des revendications 1 à 7, vecteur selon les revendications 8 à 11, ou composition selon l'une quelconque des revendications 13 à 15 à utiliser en médecine.

18. Procédé de production d'un nucléotide tel que revendiqué dans l'une quelconque des revendications 1 à 7, comprenant le fait de lier de manière opérationnelle une séquence de nucléotides codant pour une RT (à codons optimisés), un produit de troncature Nef, p17 Gag (à codons optimisés) et p24 Gag (à codons optimisés) dans l'ordre RT, Nef, p17 Gag, p24 Gag à un promoteur hétérologue.
